# EUROPÄISCHE PATENTSCHRIFT

(11) **EP 2 809 653 B1**
(45) Veröffentlichungstag und Bekanntmachung des Hinweises auf die Patenterteilung: **16.08.2017**
(21) Anmeldenummer: 13701669.7
(22) Anmeldetag: 31.01.2013
(51) Int. Cl.: C07D 231/14

(54) **VERFAHREN ZUR HERSTELLUNG VON 3,5-BIS(FLUORALKYL)-PYRAZOL-4-CARBONSÄURE-DERIVATEN UND 3,5-BIS(FLUORALKYL)-PYRAZOLEN**
METHOD FOR PRODUCING 3,5-BIS(FLUORALKYL)-PYRAZOL-4-CARBOXYLIC ACID DERIVATIVES AND 3,5-BIS(FLUORALKYL)-PYRAZOLES
PROCÉDÉ DE FABRICATION DE DÉRIVÉS D'ACIDE 3,5-BIS(FLUORALKYL)-PYRAZOL-4 CARBONIQUE ET 3,5-BIS(FLUORALKYL)-PYRAZOLES

(30) Priorität: 01.02.2012 EP 12356001
(43) Veröffentlichungstag der Anmeldung: 10.12.2014
(73) Patentinhaber: Bayer CropScience AG, 40789 Monheim (DE); Centre National de la Recherche Scientifique, 75794 Paris Cedex 16 (FR)
(72) Erfinder: PAZENOK, Sergii, 42699 Solingen (DE); LUI, Norbert, 51519 Odenthal (DE); VORS, Jean-Pierre, F-69110 Sainte Foy les Lyon (FR); LEROUX, Frédéric, F-67850 Herrlisheim (FR); GIORNAL, Florence, F-84860 Caderousse (FR)
(74) Vertreter: BIP Patents
(86) Internationale Anmeldenummer: PCT/EP2013/051930
(87) Internationale Veröffentlichungsnummer: WO 2013/113829

(56) Entgegenhaltungen:
- WO-A1-2005/042468
- WO-A2-2008/091594
- US-A1- 2009 042 726
- BO LIU ET AL: "Copper (II) hydroxide complexes of N-heterocyclic carbenes and catalytic oxidative amination of arylboronic acids", ORGANOMETALLICS, ACS, WASHINGTON, DC, US, Bd. 29, Nr. 6, 22. März 2010 (2010-03-22), Seiten 1457-1464, XP002663340, ISSN: 0276-7333, DOI: 10.1021/OM100009U [gefunden am 2010-02-23]
- SLOOP J C ET AL: "Synthesis of fluorinated heterocycles", JOURNAL OF FLUORINE CHEMISTRY, ELSEVIER, NL, Bd. 118, Nr. 1-2, 1. Dezember 2002 (2002-12-01), Seiten 135-147, XP004393618, ISSN: 0022-1139, DOI: 10.1016/S0022-1139(02)00221-X
- ITURRINO L ET AL: "Synthesis, cytostatic and trichomonacide activities of 3,5-bis-(halomethyl)pyrazoles", EUROPEAN JOURNAL OF MEDICINAL CHEMISTRY, EDITIONS SCIENTIFIQUE ELSEVIER, PARIS, FR, Bd. 22, Nr. 5, 1. September 1987 (1987-09-01), Seiten 445-451, XP023871045, ISSN: 0223-5234, DOI: 10.1016/0223-5234(87)90034-1 [gefunden am 1987-09-01]
- IGOR I. GERUS ET AL: ""Reported, but Still Unknown." A Closer Look into 3,4-Bis- and 3,4,5-Tris(trifluoromethyl)pyrazoles", THE JOURNAL OF ORGANIC CHEMISTRY, Bd. 77, Nr. 1, 6. Januar 2012 (2012-01-06) , Seiten 47-56, XP055026674, ISSN: 0022-3263, DOI: 10.1021/jo202305c
- A. BECKER: "Zur Wanderung von Brom in beta-Ketosäureestern und 1,3-Diketonen und über Versuche zur Darstellung eines Cyclopentan-1,3-dions. 1. Mitteilung", HELVETICA CHIMICA ACTA, Bd. 32, Nr. 3, 1. Januar 1949 (1949-01-01) , Seiten 1114-1122, XP055026568, ISSN: 0018-019X, DOI: 10.1002/hlca.19490320368
- MICHAEL D. THREADGILL ET AL: "The reaction of 1,1,1,5,5,5-hexafluoropentane-2,4-dione with hydrazines: a re-investigation", JOURNAL OF FLUORINE CHEMISTRY, Bd. 65, Nr. 1-2, 1. November 1993 (1993-11-01), Seiten 21-23, XP055026673, ISSN: 0022-1139, DOI: 10.1016/S0022-1139(00)80467-4
- YU H B ET AL: "A convenient synthesis of 3-polyfluoroalkyl pyrazoles and 6-polyfluoroalkyl pyrimidines from beta-polyfluoroalkyl enaminones", JOURNAL OF FLUORINE CHEMISTRY, ELSEVIER, NL, Bd. 84, Nr. 1, 31. August 1997 (1997-08-31), Seiten 65-67, XP027447290, ISSN: 0022-1139, DOI: 10.1016/S0022-1139(97)00027-4 [gefunden am 1997-08-31]
- HAROLD WEINGARTEN ET AL: "Reactions of .beta.-dicarbonyl compounds with tetrakis(dimethylamino)titanium", THE JOURNAL OF ORGANIC CHEMISTRY, Bd. 33, Nr. 4, 1. April 1968 (1968-04-01), Seiten 1506-1508, XP055026528, ISSN: 0022-3263, DOI: 10.1021/jo01268a042

## Beschreibung

Die vorliegende Erfindung betrifft ein Verfahren zur Herstellung von 3,5-Bis(fluoralkyl)-pyrazol-4-carbonsäure-Derivaten und 3,5-Bis(fluoralkyl)-pyrazolen.

Polyfluroalkylpyrazolylcarbonsäure-Derivate und 3,5-Bis(fluoralkyl)-pyrazole sind wertvolle Vorstufen von fungiziden Wirkstoffen (vgl. WO 03/070705 und WO 2008/013925).

Pyrazolcarbonsäure-Derivate werden üblicherweise hergestellt, indem man Acrylsäure-Derivate, die zwei Abgangsgruppen aufweisen, mit Hydrazinen umsetzt (vgl. WO 2009/112157 und WO 2009/106230). WO 2005/042468 offenbart ein Verfahren zur Herstellung von 2-Dihalogenacyl-3-amino-acrylsäureestern durch Umsetzung von Säurehalogeniden mit Dialkylaminoacrylsäureestern und deren anschließender Cyclisierung mit Alkylhydrazinen. WO 2008/022777 beschreibt ein Verfahren zur Herstellung von 3-Dihalomethyl-pyrazol-4-carbonsäure-Derivaten durch Umsetzung von α,α-Difluoraminen in Gegenwart von Lewissäuren mit Acrylsäurederivaten und deren anschließender Umsetzung mit Alkylhydrazinen. Die Herstellung von Pyrazolcarbonsäure-Derivaten durch Cyclisierung von Diacetessigsäure-Ethylestern bzw. deren Enolestern mit Hydrazinen ist ebenfalls bereits bekannt (Gerus et al., THE JOURNAL OF ORGANIC CHEMISTRY (2012), Bd. 77, Nr. 1, 47-56; A. Becker, HELVETICA CHIMICA ACTA (1949), Bd. 32, Nr. 3, 1114-1122). 3-Polyfluoroalkylpyrazole lassen sich durch Umsetzung von β-Polyfluoroalkylenaminonen mit Hydrazin erhalten (Yu et al., JOURNAL OF FLUORINE CHEMISTRY (1997), Bd. 84, Nr. 1, 65-67 sowie WO 2005/042468).

3,5-Bis(fluoralkyl)-pyrazole werden hergestellt indem Bisperfluoralkyldiketone (z.B. 1,1,1,5,5,5-Hexafluoracetylaceton) mit Hydrazinen umgesetzt werden (vgl. Pashkevich et al., Zhurnal Vsesoyuznogo Khimicheskogo Obshchestva im. D. I. Mendeleeva (1981), 26(1), 105-7; Threadgill et al., JOURNAL OF FLUORINE CHEMISTRY (1993), Bd. 65, Nr. 1-2, 21-23), wobei die Ausbeute lediglich 27 - 40 % beträgt. Die Synthese, die Isolierung und die Aufreinigung der Polyfluoralkyldiketonen ist sehr aufwendig, da die Verbindungen in der Regel sehr flüchtig und hoch toxisch sind. 3,5-Bisperfluoralkylpyrazol-4-carbonsäureester sind nicht bekannt.

Im Lichte des zuvor beschriebenen Standes der Technik liegt der vorliegenden Erfindung die Aufgabe zugrunde, ein Verfahren zur Verfügung zu stellen, dass die zuvor genannten Nachteile nicht aufweist und somit einen regioselektiven Zugang zu 3,5-Bis(fluoralkyl)-pyrazol-4-carbonsäure-Derivaten und 3,5-bis(fluoralkyl)-pyrazolen in hohen Ausbeuten gewährt.

Die zuvor beschriebene Aufgabe wurde gelöst durch ein Verfahren zur Herstellung von 3,5-Bis(fluoralkyl)-pyrazolen der Formel (Ia) sowie (Ib), 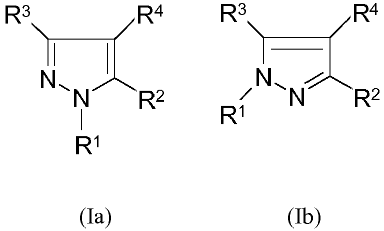 in welcher
- R¹: ausgewählt ist aus der Gruppe enthaltend H, C₁₋₁₂-Alkyl, C₃₋₈-Cycloalkyl, C₆₋₁₈-Aryl, C₇₋₁₉-Aryl-alkyl oder C₇₋₁₉-Alkylaryl, CH₂CN, CH₂CX₃, CH₂COOH, CH₂COO(C₁₋₁₂)-Alkyl, und
- X: steht unabhängig voneinander für F, Cl, Br, I;
- R² und R³: unabhängig voneinander ausgewählt ist aus C₁-C₆-Halogenalkylgruppen;
- R⁴: ausgewählt ist aus der Gruppe enthaltend Hal, COOH, (C=O)OR⁵, CN und (C=O)NR⁵R⁶, wobei R⁵ und R⁶ unabhängig voneinander ausgewählt sind aus der Gruppe enthaltend C₁₋₁₂-Alkyl, C₃₋₈-Cycloalkyl, C₆₋₁₈-Aryl-, C₇₋₁₉-Arylalkyl oder C₇₋₁₉-Alkylaryl oder wobei R⁵ und R⁶ gemeinsam mit dem N-Atom an das sie geknüpft sind einen fünf oder sechsgliedrigen Ring bilden können;
dadurch gekennzeichnet, dass in Schritt A) die Umsetzung von α, α -Dihalogenaminen der Formel (II) 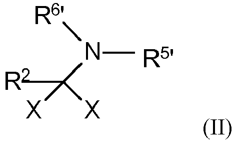 in welcher X für Cl oder F steht, und R⁵' und R⁶' unabhängig voneinander ausgewählt sind aus der Gruppe enthaltend C₁₋₆-Alkyl,
mit Verbindungen der Formel (III), 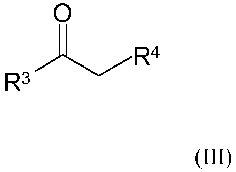 in welcher die Reste R² und R³ die oben angegebene Bedeutung haben, erfolgt und in Schritt B) die Umsetzung mit Hydrazinen der Formel (IV) 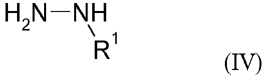 in welcher R¹ die oben angegebene Bedeutung hat, erfolgt.

Überraschenderweise lassen sich die Pyrazole der Formel (I) unter den erfindungsgemäßen Bedingungen mit guten Ausbeuten, Regioselektivitäten und in hoher Reinheit herstellen, womit das erfindungsgemäße Verfahren die oben genannten Nachteile der im Stand der Technik vorbeschriebenen Herstellungsverfahren überwindet.

Bevorzugt ist das erfindungsgemäße Verfahren, in welchem die Reste der Verbindung der Formel (Ia) und (Ib) folgend Bedeutung haben:
- R¹: ist ausgewählt aus der Gruppe enthaltend H, C₁₋₁₂-Alkyl, CH₂CN, CH₂COO-(C₁₋₁₂)-Alkyl, und
- R² und R³: sind unabhängig voneinander ausgewählt aus der Gruppe enthaltend CF₃, CF₂H, CF₂Cl;
- R⁴: ist ausgewählt aus der Gruppe enthaltend COOH, (C=O)OR⁵, CN und (C=O)NR⁵R⁶, wobei R⁵ und R⁶ unabhängig voneinander ausgewählt sind aus der Gruppe enthaltend C₁₋₁₂-Alkyl, C₃₋₈-Cycloalkyl, C₆₋₁₈-Aryl-, C₇₋₁₉-Arylalkyl oder C₇₋₁₉-Alkylaryl oder wobei R⁵ und R⁶ gemeinsam mit dem N-Atom an das sie geknüpft sind einen fünf oder sechsgliedrigen Ring bilden können.

Besonders bevorzugt ist das erfindungsgemäße Verfahren, in welchem die Reste der Verbindung der Formel (Ia) und (Ib) folgend Bedeutung haben:
- R¹: ist ausgewählt ist aus der Gruppe enthaltend H, CH₃, CH₂COO-(C₁₋₁₂)-Alkyl, und
- R² und R³: sind unabhängig voneinander ausgewählt aus der Gruppe enthaltend CF₃, CF₂H, CF₂Cl;
- R⁴: ist ausgewählt aus der Gruppe enthaltend COOH, (C=O)OR⁵.

### Allgemeine Definitionen

Im Zusammenhang mit der vorliegenden Erfindung umfasst der Begriff Halogene (**Hal**), soweit nicht anders definiert, solche Elemente, die ausgewählt sind aus der Gruppe enthaltend Fluor, Chlor, Brom und Iod, bevorzugt Fluor, Chlor und Brom, besonders bevorzugt Fluor und Chlor.

Gegebenenfalls substituierte Gruppen können einfach oder mehrfach substituiert sein, wobei bei Mehrfachsubstitutionen die Substituenten gleich oder verschieden sein können.

**Halogenalkyl:** geradkettige oder verzweigte Alkylgruppen mit 1 bis 6 und bevorzugt 1 bis 3 Kohlenstoffatomen (wie vorstehend genannt), wobei in diesen Gruppen teilweise oder vollständig die Wasserstoffatome durch Halogenatome wie vorstehend genannt ersetzt sein können, z.B. (aber nicht beschränkt auf) C₁-C₃-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor,2-difluorethyl, 2,2-Dichlor-2-fluorethyl, 2,2,2-Trichlorethyl, Pentafluorethyl und 1,1,1-Trifluorprop-2-yl. Diese Definition gilt auch für Halogenalkyl als Bestandteil eines zusammengesetzen Substitutenten wie z.B. Halogenalkylaminoalkyl etc. sofern an anderer Stelle nicht definiert. Bevorzugt sind mit einem oder mehreren Halogenatomen substituierte Alkyl-Gruppen wie beispielsweise Trifluormethyl (CF₃), Difluormethyl (CHF₂), CF₃CH₂, CF₂Cl oder CF₃CCl₂.

**Alkyl**-Gruppen sind im Zusammenhang mit der vorliegenden Erfindung, soweit nicht abweichend definiert, lineare, verzweigte oder ringförmige gesättigte Kohlenwasserstoff-Gruppen. Die Definition C₁-C₁₂-Alkyl umfasst den größten hierin definierten Bereich für einen Alkyl-Gruppe. Im Einzelnen umfasst diese Definition beispielsweise die Bedeutungen Methyl, Ethyl, n-, iso-Propyl, n-, iso-, sec- und t-Butyl, n-Pentyl, n-Hexyl, 1,3-Dimethylbutyl, 3,3-Dimethylbutyl, n-Heptyl, n-Nonyl, n-Decyl, n-Undecyl oder n-Dodecyl.

**Alkenyl**-Gruppen sind im Zusammenhang mit der vorliegenden Erfindung, soweit nicht abweichend definiert, lineare, verzweigte oder ringförmige Kohlenwasserstoff-Gruppen, die wenigstens eine einfache Unsättigung (Doppelbindung) enthalten. Die Definition C₂-C₁₂-Alkenyl umfasst den größten hierin definierten Bereich für einen Alkenyl-Gruppe. Im Einzelnen umfasst diese Definition beispielsweise die Bedeutungen Vinyl, Allyl (2-Propenyl), Isopropenyl (1-Methylethenyl), But-1-enyl (Crotyl), But-2-enyl, But-3-enyl; Hex-1-enyl, Hex-2-enyl, Hex-3-enyl, Hex-4-enyl, Hex-5-enyl; Hept-1-enyl, Hept-2-enyl, Hept-3-enyl, Hept-4-enyl, Hept-5-enyl, Hept-6-enyl; Oct-1-enyl, Oct-2-enyl, Oct-3-enyl, Oct-4-enyl, Oct-5-enyl, Oct-6-enyl, Oct-7-enyl; Non-1-enyl, Non-2-enyl, Non-3-enyl, Non-4-enyl, Non-5-enyl, Non-6-enyl, Non-7-enyl, Non-8-enyl; Dec-1-enyl, Dec-2-enyl, Dec-3-enyl, Dec-4-enyl, Dec-5-enyl, Dec-6-enyl, Dec-7-enyl, Dec-8-enyl, Dec-9-enyl; Undec-1-enyl, Undec-2-enyl, Undec-3-enyl, Undec-4-enyl, Undec-5-enyl, Undec-6-enyl, Undec-7-enyl, Undec-8-enyl, Undec-9-enyl, Undec-10-enyl, Dodec-1-enyl, Dodec-2-enyl, Dodec-3-enyl, Dodec-4-enyl, Dodec-5-enyl, Dodec-6-enyl, Dodec-7-enyl, Dodec-8-enyl, Dodec-9-enyl, Dodec-10-enyl, Dodec-11-enyl; Buta-1,3-dienyl oder Penta-1,3-dienyl.

**Alkinyl**-Gruppen sind im Zusammenhang mit der vorliegenden Erfindung, soweit nicht abweichend definiert, lineare, verzweigte oder ringförmige Kohlenwasserstoff-Gruppen, die wenigstens eine zweifache Unsättigung (Dreifachbindung) enthalten. Die Definition C₂-C₁₂-Alkinyl umfasst den größten hierin definierten Bereich für einen Alkinyl-Gruppe. Im Einzelnen umfasst diese Definition beispielsweise die Bedeutungen Ethinyl (Acetylenyl); Prop-1-inyl und Prop-2-inyl.

**Cycloalkyl:** monocyclische, gesättigte Kohlenwasserstoffgruppen mit 3 bis 8 und bevorzugt 3 bis 6 Kohlenstoffringgliedern, z.B. (aber nicht beschränkt auf) Cyclopropyl, Cyclopentyl und Cyclohexyl. Diese Definition gilt auch für Cycloalkyl als Bestandteil eines zusammengesetzen Substitutenten wie z.B. Cycloalkylalkyl etc. sofern an anderer Stelle nicht definiert;

**Aryl**-Gruppen sind im Zusammenhang mit der vorliegenden Erfindung, soweit nicht abweichend definiert, aromatische Kohlenwasserstoff-Gruppen, die ein, zwei oder mehrere Heteroatome, die ausgewählt sind aus O, N, P und S, aufweisen können. Die Definition C₆₋₁₈-Aryl umfasst den größten hierin definierten Bereich für eine Aryl-Gruppe mit 5 bis 18 Gerüst-Atomen, wobei die C-Atome gegen Heteroatome ausgetauscht sein können. Im Einzelnen umfasst diese Definition beispielsweise die Bedeutungen Phenyl, Cycloheptatrienyl, Cyclooctatetraenyl, Naphthyl und Anthracenyl; 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5- Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl; 1-Pyrrolyl, 1-Pyrazolyl, 1,2,4-Triazol-1-yl, 1-Imidazolyl, 1,2,3-Triazol-1-yl, 1,3,4-Triazol-1-yl; 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl.

**Arylalkyl**-Gruppen (Aralkyl-Gruppen) sind im Zusammenhang mit der vorliegenden Erfindung, soweit nicht abweichend definiert, durch Aryl-Gruppen substituierte Alkyl-Gruppen, die eine C₁₋₈-Alkylenkette aufweisen können und im Arylgerüst ein oder mehrere Heteroatome, die ausgewählt sind aus O, N, P und S, aufweisen können. Die Definition C₇₋₁₉-Aralkyl-Gruppe umfasst den größten hierin definierten Bereich für einen Arylalkyl-Gruppe mit insgesamt 7 bis 19 Atomen in Gerüst und Alkylenkette. Im Einzelnen umfasst diese Definition beispielsweise die Bedeutungen Benzyl und Phenylethyl.

**Alkylaryl**-Gruppen (Alkaryl-Gruppen) sind im Zusammenhang mit der vorliegenden Erfindung, soweit nicht abweichend definiert, durch Alkyl-Gruppen substituierte Aryl-Gruppen, die eine C₁₋₈-Alkylenkette aufweisen können und im Arylgerüst ein oder mehrere Heteroatome, die ausgewählt sind aus O, N, P und S, aufweisen können. Die Definition C₇₋₁₉-Alkylaryl-Gruppe umfasst den größten hierin definierten Bereich für einen Alkylaryl-Gruppe mit insgesamt 7 bis 19 Atomen in Gerüst und Alkylenkette. Im Einzelnen umfasst diese Definition beispielsweise die Bedeutungen Tolyl-, 2,3-, 2,4-, 2,5-, 2,6-, 3,4- oder 3,5-Dimethylphenyl.

Der im Zusammenhang mit der vorliegenden Erfindung verwendete Begriff *Zwischenprodukt* beschreibt die im erfindungsgemäßen Verfahren auftretenden Stoffe, die für die chemische Weiterverarbeitung hergestellt und hierbei verbraucht oder verwendet werden, um in einen anderen Stoff umgewandelt zu werden. Die Zwischenprodukte können oftmals isoliert und zwischengelagert werden oder ohne vorherige Isolierung in den darauf folgenden Reaktionsschritt eingesetzt werden. Der Begriff Zwischenprodukt umfasst auch die im allgemeinen instabilen und kurzlebigen Intermediate, die bei mehrstufigen Reaktionen (Stufenreaktionen) vorübergehend auftreten und denen lokale Minima im Energieschema des Reaktionsverlaufs zugeordnet werden können.

Die erfindungsgemäßen Verbindungen können gegebenenfalls als Mischungen verschiedener möglicher isomerer Formen, insbesondere von Stereoisomeren, wie z.B. E- und Z-, threo- und erythro-, sowie optischen Isomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen offenbart und beansprucht.

Das Verfahren wird in Schema 1 abgebildet:

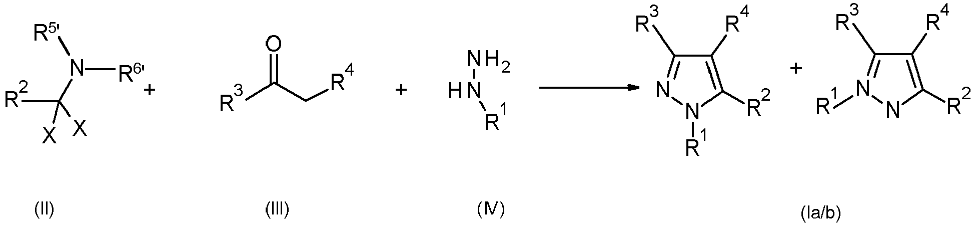

Das erfindungsgemäße Verfahren dient zur Herstellung von 3,5-Bis(fluoralkyl)-pyrazolen der Formel (Ia) oder (Ib) 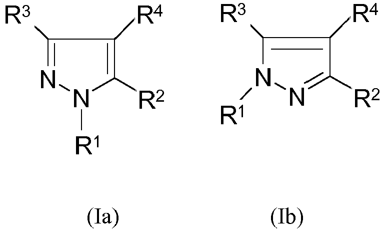 in welcher
- R¹: ausgewählt ist aus H, C₁₋₁₂-Alkyl, C₃₋₈-Cycloalkyl, C₆₋₁₈-Aryl, C₇₋₁₉-Arylalkyl oder C₇₋₁₉-Alkylaryl, CH₂CN, CH₂CX₃, CH₂COOH, CH₂COO-(C₁₋₁₂)-Alkyl;
X steht unabhängig voneinander für F, Cl, Br, I;
- R² und R³: ausgewählt ist aus C₁-C₆-Haloalkylgruppen,
- R⁴: ausgewählt ist aus der Gruppe F, Cl, Br, COOH, (C=O)OR⁵, CN und (C=O)NR⁵R⁶, wobei R⁵ und R⁶ unabhängig voneinander ausgewählt sind aus der Gruppe enthaltend C₁₋₁₂-Alkyl, C₃₋₈-Cycloalkyl, C₆₋₁₈-Aryl-, C₇₋₁₉-Arylalkyl oder C₇₋₁₉-Alkylaryl oder wobei R⁵ und R⁶ gemeinsam mit dem N-Atom an das sie geknüpft sind einen fünf oder sechsgliedrigen Ring bilden können.

In einer *bevorzugten* Ausführungsform der vorliegenden Erfindung haben die Reste in Formel (Ia) und (Ib) die folgenden Bedeutungen:
- R¹: ist ausgewählt aus H, Methyl, -CH₂COOH, CH₂COOR⁵, CH₂CN, CH₂CX₃:
X steht unabhängig voneinander für F, Cl;
- R² und R³: sind ausgewählt aus Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor,2-difluorethyl, 2,2-Dichlor-2-fluorethyl, 2,2,2-Trichlorethyl, Pentafluorethyl und 1,1,1-Trifluorprop-2-yl;
- R⁴: ist ausgewählt aus der Gruppe enthaltend Br, COOCH₃, COOEt, COOC₃H₇, CN und CONMe₂, CONEt₂.

In einer *besonders bevorzugten* Ausführungsform der vorliegenden Erfindung haben die Reste in Formel (Ia) und (Ib) die folgenden Bedeutungen:
- R¹: ist ausgewält aus H, CH₂COOH, CH₂COOMe, CH₂CN,
- R² und R³: sind ausgewählt aus der Gruppe bestehend aus Trifluormethyl, Difluormethyl, Difluorchlormethyl, Pentafluoroethyl;
- R⁴: ist ausgewählt aus der Gruppe bestehend aus Br, COOH.

Ganz besonders bevorzugt sind Verbindungen der allgemeinen Formel (I) in welcher
R1= H; R²=R³= CF₂H und R⁴ = COOEt ist oder
R¹=H; R²=R³= CF₂H und R⁴ = COOH ist oder
R¹ = CH₂COOEt, R²=R³= CF₂H und R⁴ =COOEt ist.

### Verfahrensbeschreibung

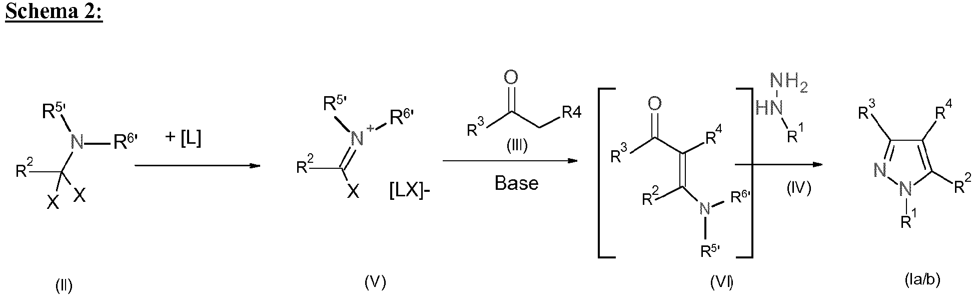

In einer Ausführungsform des erfindungsgemäßen Verfahrens werden zuerst in Schritt A) α,α-Dihalogenaminen der Formel (II), gegebenenfalls in Gegenwart einer Lewis Säure [L], mit Verbindungen der Formel (III) umgesetzt.

Bevorzugte Verbindungen der allgemeinen Formel (II) sind 1,1,2,2-Tetrafluorethyl-N,N-dimethylamin (TFEDMA), 1,1,2,2-Tetrafluorethyl-N,N-diethylamin, 1,1,2-Trifluor-2-(trifluormethyl)ethyl-N,N-dimethylamin, 1,1,2-Trifluor-2-(trifluormethyl)ethyl-N,N-diethylamin (Ishikawa-Reagenz), 1,1,2-Trifluor-2-chlor-ethyl-N,N-dimethylamin und 1,1,2-Trifluor-2-chlor-ethyl-N,N-diethylamin (Yarovenko-Reagenz).

Verbindungen der allgemeinen Formel (II) werden als Aminoalkylierungsmittel eingesetzt. Dabei sind 1,1,2,2-Tetrafluorethyl-N,N-dimethylamin (TFEDMA) und 1,1,2,2-Tetrafluorethyl-N,N-diethylamin bevorzugt und 1,1,2,2-Tetrafluorethyl-N,N-dimethylamin besonders bevorzugt. α,α-Dihalogenamine wie TFEDMA und Ishikawa-Reagenz sind kommerziell erhältlich oder können hergestellt werden (vgl. Yarovenko et al., Zh. Obshch. Khim. 1959, 29, 2159, Chem. Abstr. 1960, 54, 9724h oder Petrov et al., J. Fluor. Chem. 109 (2011) 25-31).

Von Yagupolskii et al. (Zh. Organicheskoi Khim. (1978), 14(12), 2493-6) wird gezeigt, dass die Umsetzung des Yarovenko Reagenzes (FClCHCF₂NEt₂) mit Nitrilen der Formel RCH₂CN (R = CN, CO₂Et) die Derivate der Formel (NC)RC=C(NEt₂)CHFCl in ca. 70 % Ausbeute liefert. Ketoverbindungen der Formel (III) reagieren unter diese Bedingung mit α,α-Dihalogenaminen der Formel (II) nicht.

Von Petrov et al. (J. of Fluorine Chem. (2011), 132(12), 1198-1206) wird gezeigt, dass TFEDMA (HCF₂CF₂NMe₂) mit cyclischen β-Diketonen reagiert und dabei eine Difluoracetylgruppe überträgt.

In einer bevorzugten Ausführungsform des erfindungsgemäßen Verfahrens wird das α,α-Dihalogenamin zuerst mit Lewis Säure [L], wie z.B. BF₃, AlCl₃, SbCl₅, SbF₅, ZnCl₂, umgesetzt und dann das Gemisch aus der Verbindung der Formel (III) und einer Base, in Substanz oder gelöst in einem geeigneten Lösungsmittel, zugegeben (vgl. WO 2008/022777).

Die Umsetzung von α,α-Dihalogenaminen mit Lewis Säuren (Herstellung der Imminium Salzen der Formel (V)) erfolgt entsprechend der Lehre der WO 2008/022777. Die Umsetzung erfolgt erfindungsgemäß bei Temperaturen von -20°C bis +40°C, bevorzugt bei Temperaturen von -20°C bis +30°C, besonders bevorzugt bei -10 bis 20°C und unter Normaldruck. Aufgrund der Hydrolyseempfindlichkeit der α,α-Dihalogenaminen, wird die Umsetzung in wasserfreien Apparaturen unter Inertgasatmosphäre durchgeführt.

Die Reaktionszeit ist nicht kritisch und kann, in Abhängigkeit von der Ansatzgröße und Temperatur, in einem Bereich zwischen wenigen Minuten und mehreren Stunden gewählt werden.

Erfindungsgemäß wird 1 Mol der Lewis Säure [L] mit äquimolaren Mengen der α,α-Dihalogenamin der Formel (II) umgesetzt.

Die Aminoalkylierung (Umsetzung mit Verbindung der Formel (II)) erfolgt bevorzugt in Gegenwart einer Base. Bevorzugt sind organische Basen wie Trialkylamine, Pyridine, Alkylpyridine, Phosphazene und 1,8-Diazabicyclo[5.4.0]undecen (DBU); Alkalimetallhydroxide wie z.B. Lithium-, Natrium- oder Kaliumhydroxid, Alkalimetallalcarobonate (Na₂CO₃, K₂CO₃) und Alkoholate, wie z.B. NaOMe, NaOEt, NaOt-Bu, KOt-Bu oder KF.

Für das erfindungsgemäße Verfahren werden 1 bis 5 Mol, bevorzugt 1,5 bis 4 Mol, besonders bevorzugt 2 bis 3,5 Mol der Base für die Verbindung der Formel (III) mit äquimolaren Mengen der α,α-Dihalogenamin der Formel (II) umgesetzt.

Bevorzugt werden Ketoverbindungen der Formel (III) verwendet, die ausgewählt sind aus der Gruppe enthaltend Ethyl-4,4,4-trifluoro-3-oxobutanoate, Methyl-4,4,4-trifluoro-3-oxobutanoate, Ethyl-4,4-difluoro-3-oxobutanoate, Ethyl-4-chloro-4,4-difluoro-3-oxobutanoate, 1,1,1-Trifluoraceton oder 4-Chloro-4,4-difluoro-3-oxobutanenitrile.

Geeignete Lösungsmittel sind beispielsweise aliphatische, alicyclische oder aromatische Kohlenwasserstoffe, wie z.B. Petrolether, n-Hexan, n-Heptan, Cyclohexan, Methylcyclohexan, Benzol, Toluol, Xylol oder Decalin und halogenierte Kohlenwasserstoffe, wie z.B. Chlorbenzol, Dichlorbenzol, Dichlormethan, Chloroform, Tetrachlormethan, Dichlorethan oder Trichlorethan, Ether, wie Diethylether, Diisopropylether, Methyl-tert-butylether, Methyl-tert-amylether, Dioxan, Tetrahydrofuran, 1,2-Dimethoxyethan, 1,2-Diethoxyethan oder Anisol; Nitrile, wie Acetonitril, Propionitril, n- oder iso-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Sulfoxide, wie Dimethylsulfoxid oder Sulfone, wie Sulfolan. Besonders bevorzugt sind beispielsweise THF, Acetonitrile, Ether, Toluol, Xylol, Chlorbenzol, n-Hexan, Cyclohexan oder Methylcyclohexan, ganz besonders bevorzugt sind beispielsweise Acetonitrile, THF, Ether oder Dichlormethan.

Die gebildeten Zwischenprodukte der Formel (VI) können ohne vorherige Aufarbeitung in den Cyclisierungsschritt mit Hydrazinen eingesetzt werden.

Alternativ können die Zwischenprodukte durch geeignete Aufarbeitungsschritte und gegebenenfalls weiterer Reinigung isoliert und charakterisiert werden.

### Schritt B) Cyclisierung

Die Cyclisierung in Schritt B) durch Umsetzung mit Verbindung (IV) erfolgt im erfindungsgemäßen Verfahren bei Temperaturen von -40°C bis +80°C, bevorzugt bei Temperaturen von -10°C bis +60 °C, besonders bevorzugt bei -10 bis 50°C und unter Normaldruck.

Die Reaktionszeit ist nicht kritisch und kann in Abhängigkeit von der Ansatzgröße in einem größeren Bereich gewählt werden.

Üblicherweise erfolgt der Cylisierungsschritt B) ohne Lösemittelwechsel.

Erfindungsgemäß werden 1 bis 2 Mol, bevorzugt 1 bis 1,5 der Hydrazine der Formel (IV) für 1 Mol der Verbindung der Formel (III) eingesetzt.

Bevorzugt werden alle Reaktionsschritte des erfindungsgemäßen Verfahrens im selben Lösungsmittel durchgeführt. Im Zusammenhang mit der vorliegenden Erfindung werden beispielsweise Hydrazinhydrat, Methylhydrazine, Ethylhydrazine, Phenylhydrazine, *tert*-Butylhydrazine, Methyl- oder Ethylhydrazinoacetatehydrochloride oder Hydrazinoacetonitrilehydrochlorid eingesetzt.

Die genannten Hydrazine der Formel (IV) sind kommerziell erhältlich oder können wie zum Beispiel in Niedrich et al, Journal fuer Praktische Chemie (Leipzig) (1962), 17 273-81. Carmi, A.; Pollak, Journal of Organic Chemistry (1960), 25 44-46 hergestellt werden.

Geeignete Lösungsmittel sind beispielsweise aliphatische, alicyclische oder aromatische Kohlenwasserstoffe, wie z.B. Petrolether, n-Hexan, n-Heptan, Cyclohexan, Methylcyclohexan, Benzol, Toluol, Xylol oder Decalin, und halogenierte Kohlenwasserstoffe, wie z.B. Chlorbenzol, Dichlorbenzol, Dichlormethan, Chloroform, Tetrachlormethan, Dichlorethan oder Trichlorethan, Ether, wie Diethylether, Diisopropylether, Methyl-tert-butylether, Methyl-tert-amylether, Dioxan, Tetrahydrofuran, 1,2-Dimethoxyethan, 1,2-Diethoxyethan oder Anisol; Alkohole wie Metanol, Ethanol, Isopropanol oder Buthanol, Nitrile, wie Acetonitril, Propionitril, n- oder iso-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Sulfoxide, wie Dimethylsulfoxid oder Sulfone, wie Sulfolan. Besonders bevorzugt sind beispielsweise AcetonitrileToluol, Xylol, Chlorbenzol, n-Hexan, Cyclohexan oder Methylcyclohexan, ganz besonders bevorzugt sind beispielsweise Acetonitrile, THF, Toluol, Xylol. Nach beendeter Reaktion wird beispielsweise die Lösemitteln entfernt und das Produkt durch Filtration isoliert oder zunächst das Produkt mit Wasser gewaschen, extrahiert, die organische Phase abgetrennt und das Lösungsmittel in Vakuum entfernt.

Die Verbindungen der Formel (I) mit R⁴ = COOR⁵ können dann zu Pyrazolsäuren der Formel (I) R⁴=COOH umgewandelt werden.

Die Umwandlung wird in der Regel unter sauren oder basischen Bedingungen durchgeführt.

Für saure Hydrolyse bevorzugt sind mineralische Säuren, beispielsweise H₂SO₄, HCl, HSO₃Cl, HF, HBr, HI, H₃PO₄ oder organische Säuren, beispielsweise CF₃COOH, p-Toluolsulfonsäure, Methansulfonsäure, Trifluormethansulfonsäure. Die Reaktion kann durch der Zusatz von Katalysatoren wie beispielsweise FeCl₃, AlCl₃, BF₃, SbCl₃, NaH₂PO₄ beschleunigt werden. Die Reaktion kann ebenfalls ohne Zusatz von Säure nur in Wasser durchgeführt werden.

Basische Hydrolyse erfolgt in Gegenwart von anorganischen Basen wie Alkalimetallhydroxide wie z.B. Lithium-, Natrium- oder Kaliumhydroxid, Alkalimetallcarbonate, wie beispielsweise Na₂CO₃, K₂CO₃ und Alkalimetall-Acetate wie beispielsweise NaOAc, KOAc, LiOAc, sowie Alkalimetall-Alkoholate, wie z.B. NaOMe, NaOEt, NaOt-Bu, KOt-Bu von organischen Basen wie Trialkylamine, Alkylpyridine, Phosphazene und 1,8-Diazabicyclo[5.4.0]undecen (DBU). Bevorzugt sind die anorganische Basen wie z.B. NaOH, KOH, Na₂CO₃ oder K₂CO₃.

Bevorzugt erfolgt die Umwandlung mittels basischer Hydrolyse.

Die Durchführung des erfindungsgemäßen Verfahrensschritts erfolgt bevorzugt innerhalb eines Temperaturbereichs von 20°C bis +150°C, besonders bevorzugt bei Temperaturen von 30°C bis +110 °C, ganz besonders bevorzugt bei 30 bis 80°C.

Der erfindungsgemäße Verfahrensschritt wird im Allgemeinen unter Normaldruck durchgeführt. Alternativ ist es jedoch auch möglich, im Vakuum oder unter Überdruck (z.B. Umsetzung im Autoklav mit wässriger HCl) zu arbeiten.

Die Reaktionszeit kann, in Abhängigkeit von der Ansatzgröße und der Temperatur, in einem Bereich zwischen 1 Stunde und mehreren Stunden gewählt werden.

Der Reaktionsschritt kann in Substanz oder in einem Lösungsmittel durchgeführt werden. Bevorzugt wird die Reaktion in einem Lösungsmittel durchgeführt. Geeignete Lösungsmittel sind beispielsweise ausgewählt aus der Gruppe enthaltend Wasser, Alkohole wie Metanol, Ethanol, Isopropanol oder Buthanol, aliphatischen und aromatischen Kohlenwasserstoffen, wie z.B. n-Hexan, Benzol oder Toluol, die durch Fluor- und Chloratome substituiert sein können, wie Methylenchlorid, Dichlorethan, Chlorbenzol oder Dichlorbenzol; Ethern, wie z.B. Diethylether, Diphenylether Methyl-tert-butylether, Isopropylethylether, Dioxan, Diglym, Dimethylglycol, Dimethoxyethane (DME) oder THF; Nitrilen wie Methylnitril, Butylnitril oder Phenylnitril; Amide wir Dimethylformamid (DMF) oder N-methlypyrollidon (NMP) oder Mischungen solcher Lösungsmittel, wobei Wasser, Acetonitril, Dichlormethan und Alkohole (Ethanol) besonders bevorzugt sind.

Die gemäß dem erfindungsgemäßen Verfahren hergestellten Verbindungen (Ia) und (Ib) werden zur Herstellung fungizider Wirkstoffe verwendet.

Ebenfalls Gegenstand der vorliegenden Erfindung sind Verbindungen der Formel (VI) 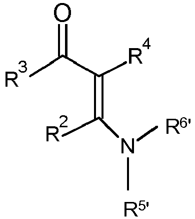 in welcher
- R² und R³: unabhängig voneinander ausgewählt sind aus der Gruppe enthaltend CF₃, CF₂H, CF₂Cl;
- R⁴: ausgewählt ist aus der Gruppe enthaltend (C=O)OR⁵;
- R⁵' und R⁶': unabhängig voneinander ausgewählt sind aus der Gruppe enthaltend C₁₋₆-Alkyl.

Das erfindungsgemäße Verfahren wird in den nachstehenden Beispielen weiter beschrieben. Die Beispiele sind jedoch nicht in einschränkender Weise zu interpretieren.

### Charakterisierung der Zwischenverbindung (YI):

### Ethyl 2-(2-chloro-2,2-difluoroacetyl)-3-(dimethylamino)-4,4-difluorobut-2-enoate:

BF₃.OEt₂ (0.12 mL, 1.0 mmol) wurde zu einer Lösung von TFEDMA (0.12 mL, 1.0 mmol) in trockenem Dichlormethan (1 mL) unter Argon in einem Teflonkolben gegeben. Die Lösung wurde 15 min bei Raumtemperatur gerührt, bevor das Dichlormethan unter Vakuum entfernt wurde. Der Rückstand wurde dann in perdeuteriertem Acetonitril (1 mL) aufgenommen. In einem zweiten Teflonkolben wurde Ethyl-4-chloro-4,4-difluoroacetoacetat (0.20 g, 1.0 mol) zu einer Lösung von Kaliumfluorid (0.18 g, 3.0 mmol) in CD3CN (2 mL) gegeben und 15 min bei Raumtemperatur gerührt. Hierzu wurde dann bei -30°C der Inhalt des ersten Teflonkolben zugetropft und die Reaktionsmischung über Nacht bei Raumtemperatur gerührt und dann durch ¹H und ¹³C NMR Spektroskopie analysiert. Die Zwischenverbindung (Ethyl 2-(2-chloro-2,2-difluoroacetyl)-3-(dimethylamino)-4,4-difluorobut-2-enoate) konnte als 2:1 Mischung (¹H NMR) in Gegenwart von Ethyl-3-(dimethylamino)-4,4-difluorobut-2-enoate charakterisiert werden.

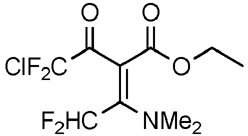

¹H NMR (CD₃CN, 300 MHz, 25 °C): *δ* = 6.36 (t, 1H, CHF₂, J_{H-F} = 53.2 Hz), 4.21 (q, 2H, CH₂, J = 7.2 Hz), 3.07 (t, 3H, NMe, J_{H-F} = 1.2 Hz), 2.95 (t, 3H, NMe, J_{H-F} = 1.2 Hz), 1.26 (t, 3H, CH₃, J = 7.2 Hz) ppm.

¹³C NMR (CD₃CN, 75 MHz, 25 °C): *δ* = 185.3 (F₂ClC-CO), 164.9 (CO), 161.7 (t, C_{IV}-NMe₂, J_{C-F} = 25.1 Hz), 119.4 (t, CF₂Cl, J_{C-F} = 304.3 Hz), 108.1 (t, CHF₂, J_{C-F} = 244.4 Hz), 98.1 (t, C_{IV}, J_{C-F} = 4.8 Hz), 61.9 (CH₂), 35.0 (N-Me₂), 13.3 (CH₃) ppm.

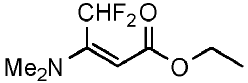

¹H NMR (CD₃CN, 300 MHz, 25 °C): *δ* = 6.65 (t, 1H, CHF₂, J_{H-F} = 51.9 Hz), 5.70 (s, 1H, CH), 4.31 (q, 2H, CH₂, J = 7.1 Hz), 3.91 (t, 3H, NMe, J_{H-F} = 0.8 Hz), 3.22 (t, 3H, NMe, J_{H-F} = 1.2 Hz), 1.31 (t, 3H, CH₃, J = 7.1 Hz) ppm.

¹³C NMR (CD₃CN, 75 MHz, 25 °C): *δ* = 171.3 (CO), 163.4 (t, C_{IV}-NMe₂, J_{C-F} = 21.3 Hz), 110.5 (t, CHF₂, J_{C-F} = 246.7 Hz), 91.1 (t, C_{IV}, J_{C-F} = 4.4 Hz), 61.2 (CH₂), 36.4 (N-Me₂), 13.3 (CH₃) ppm.

### Herstellungsbeispiele

### Beispiel 1:

### N-Methyl-3-difluoromethyl-5-trifluoromethyl-4-pyrazolcarbonsäureethylester:

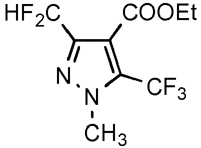

BF₃.OEt₂ (0.62 mL, 5.0 mmol) wurde zu einer Lösung von TFEDMA (0.59 mL, 5.0 mmol) in trockenem Dichlormethane (5 mL) under Argon in einem Teflonkolben gegeben. Die Lösung wurde 15 min bei Raumtemperatur gerührt, bevor das Dichlormethan unter Vakuum entfernt wurde. Der Rückstand wurde dann in trockenem Acetonitril (5 mL) aufgenommen. In einem zweiten Teflonkolben wurde Ethyl-4,4,4-trifluoroacetoacetat (0.73 mL, 5.0 mmol) zu einer Lösung von Kaliumfluoride (0.88g, 15.0 mmol) in trockenem Acetonitril (10 mL) gegeben und 15 min bei Raumtemperatur gerührt. Hierzu wurde bei -30°C der Inhalt des ersten Kolbens zugetropft. Die Reaktionsmischung wurde im Kältebad auf Raumtemperatur gebracht und über Nacht gerührt. Methylhydrazin (0.32 mL, 6.0 mmol) wurde dann bei Raumtemperatur zugetropft und die Mischung über Nacht gerührt. Das Lösemittel wurde im Vakuum entfernt und der Rückstand durch Flashchromatographie auf Kieselgel mit einer Pentane/Diethylether Mischung (9:1 - 8:2) gereinigt. *N*-Methyl-3-difluoromethyl-5-trifluoromethyl-4-pyrazolcarbonsäureethylester (0.99 g, 3.64 mmol, 73%) wurde als gelbes Öl erhalten.

¹H NMR (CDCl₃, 300 MHz, 25 °C): *δ* = 7.00 (t, 1H, CHF₂, J_{H-F} = 54 Hz), 4.37 (q, 2H, CH₂, J = 7.2 Hz), 4.12 (s, 3H, N-CH₃), 1.37 (t, 3H, CH₃, J = 7.2 Hz) ppm. ¹³C NMR (CDCl₃, 75 MHz, 25 °C): *δ* = 160.2 (CO), 145.7 (t, C_{IV}arom, J_{C-F} = 25.6 Hz), 133.2 (q, C_{IV}arom, J_{C-F} = 40.3 Hz), 119.0 (q, CF₃, J_{C-F} = 271.2 Hz), 114.4 (C_{IV}arom), 109.0 (t, CHF₂, J_{C-F} = 237.9 Hz), 61.9 (CH₂), 40.8 (q, N-CH₃, J_{C-F} = 3.2 Hz), 13.8 (CH₃) ppm. ¹⁹F NMR (CDCl₃, 282MHz, 25°C): *δ* = -57.6 (CF₃), -116.4 (CHF₂) ppm.

### Beispiel 2:

Wie Beispiel 1: Es wurde jedoch Pyridin anstatt Kaliumfluorid verwendet. Die Ausbeute beträgt 63%.

### Beispiel 3:

### N-Methyl-3-difluoromethyl-5-trifluoromethyl-4-pyrazolcarbonsäure:

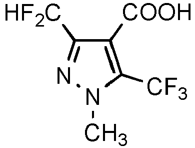

*N*-Methyl-3-difluoromethyl-5-trifluoromethyl-4-pyrazolcarbonsäureethylester (0.5g, 1.84 mmol) wurde in Ethanol (3 mL) langsam mit einer 8N wässrigen Natriumhydroxid Lösung (0.7 mL) versetzt und 3h bei Raumtemperatur gerührt. Das Lösemittel wurde abrotiert, der Rückstand in Wasser (10 mL) aufgenommen und mit Diethylether (10 mL) extrahiert. Nach Ansäuern auf pH 1 mit 1M HCl wurde mit Ethylacetat (3 x 10 mL) extrahiert. Die vereinigten organischen Phasen wurden über Natriumsulfat getrocknet, filtriert und das Lösemittel abrotiert. *N*-Methyl-3-difluoromethyl-5-trifluoromethyl-4-pyrazolcarbonsäure (0.44g, 1.80 mmol, 98%) wurde als gelblicher Feststoff isoliert.

¹H NMR (CDCl₃, 300 MHz, 25 °C): *δ* = 7.08 (t, 1H, CHF₂, J_{H-F} = 53.5 Hz), 4.16 (s, 3H, N-CH₃) ppm. ¹³C NMR (CDCl₃, 75 MHz, 25 °C): *δ* = 165.5 (CO), 146.7 (t, C_{IV}arom, J_{C-F} = 18.8 Hz), 134.4 (q, C_{IV}arom, J_{C-F} = 30.8 Hz), 118.8 (q, CF₃, J_{C-F} = 202.5 Hz), 112.9 (C_{IV}arom), 108.7 (t, CHF₂, J_{C-F} = 177.0 Hz), 41.1 (q, N-CH₃, J _{C-F} = 2.3 Hz) ppm. ¹⁹F NMR (CDCl₃, 282MHz, 25°C): *δ* = -57.9 (CF₃), -117.3 (CHF₂, J_{F-H}= 53.5 Hz) ppm.

### Beispiel 4:

### N-H-3-Difluoromethyl-5-trifluoromethyl-4-pyrazolcarbonsäureethylester:

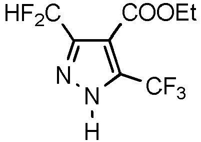

BF₃.OEt₂ (0.31 mL, 2.5 mmol) wurde zu einer Lösung von TFEDMA (0.30 mL, 2.5 mmol) in trockenem Dichlormethan (2.5 mL) unter Argon in einem Teflonkolben gegeben. Die Lösung wurde 15 min bei Raumtemperatur gerührt, bevor das Dichlormethan unter Vakuum entfernt wurde. Der Rückstand wurde dann in trockenem Acetonitril (2.5 mL) aufgenommen. In einem zweiten Teflonkolben wurde Ethyl-4,4,4-trifluoroacetoacetat (0.37 mL, 2.5 mmol) zu einer Lösung von Kaliumfluoride (0.44 g, 7.5 mmol) in trockenem Acetonitril (5 mL) gegeben und 15 min bei Raumtemperatur gerührt. Hierzu wurde bei -30°C der Inhalt des ersten Kolbens zugetropft. Die Reaktionsmischung wurde im Kältebad auf Raumtemperatur gebracht und über Nacht gerührt. Hydrazinhydrat (0.15 mL, 3.0 mmol) wurde dann bei Raumtemperatur zugetropft und die Mischung 24h gerührt. Das Lösemittel wurde im Vakuum entfernt und der Rückstand durch Flashchromatographie auf Kieselgel mit einer Pentane/Diethylether Mischung (9:1 - 7:3) gereinigt. *N*-H-3-Difluoromethyl-5-trifluoromethyl-4-pyrazolcarbonsäureethylester (0.48 g, 1.88 mmol, 75%) wurde als gelbliches Öl erhalten welches beim Stehenlassen auskristallisiert.

¹H NMR (CDCl₃, 300 MHz, 25 °C): *δ* = 11.07 (brs, 1H, NH), 7.22 (t, 1H, CHF₂, J_{H-F} = 53.5 Hz), 4.39 (q, 2H, CH₂, J = 6.9 Hz), 1.38 (t, 3H, CH₃, J = 6.9 Hz) ppm. ¹³C NMR (CDCl₃, 75 MHz, 25 °C): *δ* = 160.4 (CO), 142.2 (t, C_{IV}arom, J_{C-F} = 18.3 Hz), 142.2 (q, C_{IV}arom, J_{C-F} = 32.0 Hz), 119.7 (q, CF₃, J_{C-F} = 268.1 Hz), 111.7 (C_{IV}arom), 107.4 (t, CHF₂, J_{C-F} = 237.5 Hz), 62.0 (CH₂), 13.7 (CH₃) ppm. ¹⁹F NMR (CDCl₃, 282MHz, 25°C): *δ* = -62.5 (CF₃), -117.1 (CHF₂, J_{F-H} = 53.5 Hz) ppm.

### Beispiel 5:

Wie Beispiel 3: Es wurde jedoch Pyridin anstatt Kaliumfluorid verwendet. Die Ausbeute beträgt 67%.

### Beispiel 6:

### N-Methyl-3,5-bis(difluoromethyl)-4-pyrazolcarbonsäureethylester:

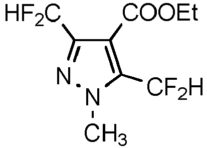

BF₃.OEt₂ (1.24 mL, 10.0 mmol) wurden zu einer Lösung von TFEDMA (1.20 mL, 10.0 mmol) in trockenem Dichlormethan (10 mL) unter Argon in einem Teflonkolben gegeben. Die Lösung wurde 15 min bei Raumtemperatur gerührt, bevor das Dichlormethan unter Vakuum entfernt wurde. Der Rückstand wurde dann in trockenem Acetonitril (10 mL) aufgenommen. In einem zweiten Teflonkolben wurde Ethyl-4,4-difluoroacetoacetat (1.03 mL, 10.0 mmol) zu einer Lösung von Pyridin (1.6 mL, 20.0 mmol) in trockenem Acetonitril (20 mL) gegeben und 15 min bei Raumtemperatur gerührt. Hierzu wurde bei -30°C der Inhalt des ersten Kolbens zugetropft. Die Reaktionsmischung wurde im Kältebad auf Raumtemperatur gebracht und über Nacht gerührt. Methylhydrazin (0.79 mL, 15.0 mmol) wurde dann bei Raumtemperatur zugetropft und die Mischung über Nacht gerührt. Das Lösemittel wurde im Vakuum entfernt und der Rückstand durch Flashchromatographie auf Kieselgel mit einer Pentane/Diethylether Mischung (10:0 - 8:2) gereinigt. (10:0 to 8:2). *N*-Methyl-3,5-difluoromethyl-4-pyrazolcarbonsäureethylester (1.75 g, 6.89 mmol, 69%) wurde als farbloses Öl erhalten welches beim Stehenlassen auskristallisiert.

¹H NMR (CDCl₃, 300 MHz, 25 °C): *δ* = 7.48 (t, 1H, CHF₂, J_{H-F} = 52.6 Hz,), 7.04 (t, 1H, CHF₂, J_{H-F} = 53.8 Hz), 4.38 (q, 2H, CH₂, J = 7.1 Hz), 4.12 (s, 3H, N-CH₃), 1.39 (t, 3H, CH₃, J = 7.2 Hz) ppm. ¹³C

NMR (CDCl₃, 75 MHz, 25 °C): *δ* = 161.1 (CO), 145.3 (t, C_{IV}arom, J_{C-F} = 24.9 Hz), 138.2 (t, C_{IV}arom, J_{C-F} = 24.1 Hz), 112.9 (m, C_{IV}arom), 109.1 (t, CHF₂, J_{C-F} = 237.6 Hz), 107.2 (t, CHF₂, J_{C-F} = 236.3 Hz), 61.5 (CH₂), 39.6 (t, N-CH₃, J _{C-F} = 3.1 Hz), 13.9 (CH₃) ppm. ¹⁹F NMR (CDCl₃, 282MHz, 25°C): *δ* = - 117.00 (CHF₂, J_{F-H} = 53.8 Hz), -117.04 (CHF₂, J_{F-H} = 52.6 Hz) ppm.

### Beispiel 7:

### N-Methyl-3,5-bis(difluoromethyl)-4-pyrazolcarbonsäure:

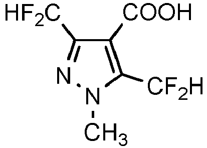

*N*-Methyl-3,5-difluoromethyl-4-pyrazolcarbonsäureethylester (0.5 g, 2.0 mmol) wurde in Ethanol (3 mL) langsam mit einer 8N wässrigen Natriumhydroxid Lösung (0.8 mL) versetzt und 2h bei Raumtemperatur gerührt. Das Lösemittel wurde abrotiert, der Rückstand in Wasser (10 mL) aufgenommen und mit Diethylether (10 mL) extrahiert. Nach Ansäuern auf pH 1 mit 6M HCl wurde mit Ethylacetat (3 x 10 mL) extrahiert. Die vereinigten organischen Phasen wurden über Natriumsulfat getrocknet, filtriert und das Lösemittel abrotiert. *N*-Methyl-3,5-difluoromethyl-4-pyrazolcarbonsäure (0.44g, 1.95 mmol, 97%) wurde als farbloser Feststoff isoliert.

¹H NMR (CDCl₃, 300 MHz, 25 °C): *δ* = 12.16 (brs, 1H, COOH), 7.48 (t, 1H, CHF₂, J_{H-F} = 52.4 Hz), 7.08 (t, 1H, CHF₂, J_{H-F} = 53.6 Hz), 4.16 (s, 3H, N-CH₃) ppm. ¹³C NMR (CDCl₃, 75 MHz, 25 °C): *δ* = 166.9 (CO), 146.4 (t, C_{IV}arom, J_{C-F} = 25.1 Hz), 139.2 (t, C_{IV}arom, J_{C-F} = 24.4 Hz), 111.5 (C_{IV}arom), 108.8 (t, CHF₂, J_{C-F} = 238.1 Hz), 106.9 (t, CHF₂, J_{C-F} = 237.0 Hz), 39.9 (t, N-CH₃, J _{C-F} = 3.1 Hz) ppm. ¹⁹F NMR (CDCl₃, 282MHz, 25°C) : *δ* = -117.1 (CHF₂, J_{F-H} = 52.6 Hz), -117.3 (CHF₂, J_{F-H} = 53.7 Hz) ppm.

### Beispiel 8:

### N-H-3,5-Bis(difluoromethyl)-4-pyrazolcarbonsäureethylester:

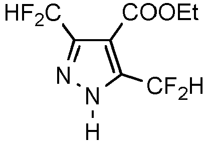

BF₃.OEt₂ (1.85 mL, 15.0 mmol) wurde zu einer Lösung von TFEDMA (1.76 mL, 15.0 mmol) in trockenem Dichlormethane (15 mL) unter Argon in einem Teflonkolben gegeben. Die Lösung wurde 15 min bei Raumtemperatur gerührt, bevor das Dichlormethan unter Vakuum entfernt wurde. Der Rückstand wurde dann in trockenem Acetonitril (15 mL) aufgenommen. In einem zweiten Teflonkolben wurde Ethyl-4,4-difluoroacetoacetat (1.55 mL, 15 mmol) zu einerr Lösung von Kaliumfluoride (2.61 g, 45 mmol) in trockenem Acetonitril (30 mL) gegeben und 15 min bei Raumtemperatur gerührt. Hierzu wurde bei -30°C der Inhalt des ersten Kolbens zugetropft. Die Reaktionsmischung wurde im Kältebad auf Raumtemperatur gebracht und über Nacht gerührt. Hydrazinhydrat (1.1 mL, 22.5 mmol) wurde dann bei Raumtemperatur zugetropft und die Mischung über Nacht gerührt. Das Lösemittel wurde im Vakuum entfernt und der Rückstand durch Flashchromatographie auf Kieselgel mit einer Pentane/Diethylether Mischung (9:1 - 7:3) gereinigt. *N-*H-3,5-Difluoromethyl-4-pyrazolcarbonsäureethylester (2.02 g, 8.40 mmol, 56%) wurde als farbloser Feststoff isoliert.

¹H NMR (CDCl₃, 300 MHz, 25 °C): *δ* = 7.15 (t, 2H, CHF₂, J_{H-F} = 53.6 Hz), 4.39 (q, 2H, CH₂, J = 7.1 Hz), 1.39 (t, 3H, CH₃, J = 7.1 Hz) ppm. ¹³C NMR (CDCl₃, 75 MHz, 25 °C): *δ* = 161.1 (CO), 143.8 (t, C_{IV}arom, J_{C-F} = 23.1 Hz), 111.6 (C_{IV}arom), 108.2 (t, CHF₂, J_{C-F} = 238.4 Hz), 61.7 (CH₂), 13.9 (CH₃) ppm. ¹⁹F NMR (CDCl₃, 282MHz, 25°C): *δ* = -117.3 (CHF₂, J_{F-H} = 53.6 Hz) ppm.

### Beispiel 9:

Wie Beispiel 8:Es wurde jedoch Pyridin anstatt Kaliumfluorid verwendet. Die Ausbeute beträgt 29 %.

### Beispiel 10:

### N-Methyl-3-difluoromethyl-5-chlorodifluoromethyl-4-pyrazolcarbonsäureethylester:

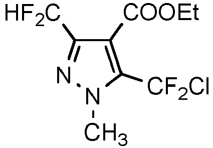

BF₃.OEt₂ (1.24 mL, 10.0 mmol) wurde zu einer Lösung von TFEDMA (1.20 mL, 10.0 mmol) in trockenem Dichlormethan (10 mL) unter Argon in einem Teflonkolben gegeben. Die Lösung wurde 15 min bei Raumtemperatur gerührt, bevor das Dichlormethan unter Vakuum entfernt wurde. Der Rückstand wurde dann in trockenem Acetonitril (10 mL) aufgenommen. In einem zweiten Teflonkolben wurde Ethyl-4-chloro-4,4-difluoroacetoacetat (2.0 g, 10.0 mmol) zu einer Lösung von Pyridin (2.42 mL, 30.0 mmol) in trockenem Acetonitril (20 mL) gegeben und 15 min bei Raumtemperatur gerührt. Hierzu wurde bei -30°C der Inhalt des ersten Kolbens zugetropft. Die Reaktionsmischung wurde im Kältebad auf Raumtemperatur gebracht und über Nacht gerührt. Methylhydrazin (0.79 mL, 15.0 mmol) wurde dann bei Raumtemperatur zugetropft und die Mischung über Nacht gerührt. Das Lösemittel wurde im Vakuum entfernt und der Rückstand durch Flashchromatographie auf Kieselgel mit einer Pentane/Diethylether Mischung (10:0 - 8:2) gereinigt. *N*-Methyl-3-difluoromethyl-5-chlorodifluoromethyl-4-pyrazolcarbonsäureethylester (2.07 g, 7.18 mmol, 72%) wurde als farblose Flüssigkeit isoliert.

¹H NMR (CDCl₃, 300 MHz, 25 °C): *δ* = 6.97 (t, 1H, CHF₂, J_{H-F} = 53.9 Hz,), 4.37 (q, 2H, CH₂, J = 7.1 Hz), 4.10 (t, 3H, N-CH₃, J_{H-F} = 2.2 Hz), 1.38 (t, 3H, CH₃, J = 7.1 Hz) ppm. ¹³C NMR (CDCl₃, 75 MHz, 25 °C): *δ* = 160.3 (CO), 145.3 (t, C_{IV}arom, J_{C-F} = 25.7 Hz), 137.5 (t, C_{IV}arom, J_{C-F} = 33.3 Hz), 119.9 (t, CF₂Cl, J_{C-F} = 288.8 Hz), 112.7 (C_{IV}arom), 109.1 (t, CHF₂, J_{C-F} = 237.8 Hz), 61.8 (CH₂), 40.6 (t, N-CH₃, J_{C-F} = 4.6 Hz), 13.7 (CH₃) ppm. ¹⁹F NMR (CDCl₃, 282MHz, 25°C): *δ* = -47.9 (CF₂Cl), -116.7 (CHF₂, J_{F-H} = 53.9 Hz) ppm.

### Beispiel 11:

### N-Methyl-3-difluoromethyl-5-chlorodifluoromethyl-4-pyrazolcarbonsäure:

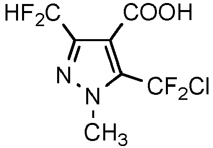

*N*-Methyl-3-difluoromethyl-5-chlorodifluoromethyl-4-pyrazolcarbonsäureethylester (0.5g, 1.73 mmol) wurde in Ethanol (3 mL) langsam mit einer 8N wässrigen Natriumhydroxid Lösung (0.7 mL) versetzt und 3h bei Raumtemperatur gerührt. Das Lösemittel wurde abrotiert, der Rückstand in Wasser (10 mL) aufgenommen und mit Diethylether (10 mL) extrahiert. Nach Ansäuern auf pH 1 mit 6M HCl wurde mit Ethylacetat (3 x 10 mL) extrahiert. Die vereinigten organischen Phasen wurden über Natriumsulfat getrocknet, filtriert und das Lösemittel abrotiert. *N*-Methyl-3-difluoromethyl-5-chlorodifluoromethyl-4-pyrazolcarbonsäure (0.36g, 1.38 mmol, 80%) wurde als farbloser Feststoff isoliert.

¹H NMR (CDCl₃, 300 MHz, 25 °C): *δ* = 12.15 (brs, 1H, COOH), 7.07 (t, 1H, CHF₂, J_{H-F} = 53.6 Hz), 4.15 (t, 3H, N-CH₃, J_{H-F} = 2.1 Hz) ppm. ¹³C NMR (CDCl₃, 75 MHz, 25 °C): *δ* = 165.8 (CO), 146.4 (t, C_{IV}arom, J_{C-F} = 25.3 Hz), 138.9 (t, C_{IV}arom, J_{C-F} = 33.6 Hz), 119.6 (t, CF₂Cl, J_{C-F} = 289.4 Hz), 111.15 (C_{IV}arom), 108.8 (t, CHF₂, J_{C-F} = 238.4 Hz), 41.0 (t, N-CH₃, J_{C-F} = 4.9 Hz) ppm. ¹⁹F NMR (CDCl₃, 282MHz, 25°C): *δ* = -48.1 (CF₂Cl), -117.2 (CHF₂, J_{F-H} = 53.6 Hz) ppm.

### Beispiel 12:

### N-H-3-Difluoromethyl-5-chlorodifluoromethyl-4-pyrazolcarbonsäureethylester:

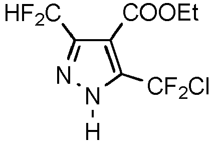

BF₃.OEt₂ (0.62 mL, 5.0 mmol) wurde zu einer Lösung von TFEDMA (0.59 mL, 5.0 mmol) in trockenem Dichlormethan (5 mL) unter Argon in einem Teflonkolben gegeben. Die Lösung wurde 15 min bei Raumtemperatur gerührt, bevor das Dichlormethan unter Vakuum entfernt wurde. Der Rückstand wurde dann in trockenem Acetonitril (5 mL) aufgenommen. In einem zweiten Teflonkolben wurde Ethyl-4-chloro-4,4-difluoroacetoacetat (1.0 g, 5.0 mmol) zu einer Lösung von Pyridin (1.19 g, 15 mmol) in trockenem Acetonitril (10 mL) gegeben und 15 min bei Raumtemperatur gerührt. Hierzu wurde bei -30°C der Inhalt des ersten Kolbens zugetropft. Die Reaktionsmischung wurde im Kältebad auf Raumtemperatur gebracht und über Nacht gerührt. Hydrazinhydrat (0.37 mL, 7.5 mmol) wurde dann bei Raumtemperatur zugetropft und die Mischung über Nacht gerührt. Das Lösemittel wurde im Vakuum entfernt und der Rückstand durch Flashchromatographie auf Kieselgel mit einer Pentane/Diethylether Mischung (9:1 - 7:3) gereinigt. *N*-H-3-Difluoromethyl-5-chlorodifluoromethyl-4-pyrazolcarbonsäureethylester (0.99 g, 3.61 mmol, 72%) wurde als leicht gelbliches Öl isoliert.

¹H NMR (CDCl₃, 300 MHz, 25 °C): *δ* = 11.62 (brs, 1H, NH), 7.25 (t, 2H, CHF₂, J_{H-F} = 53.5 Hz), 4.41 (q, 2H, CH₂, J = 7.1 Hz), 1.41 (t, 3H, CH₃, J = 7.1 Hz) ppm. ¹³C NMR (CDCl₃, 75 MHz, 25 °C): *δ* = 160.6 (CO), 146.3 (t, C_{IV}arom, J_{C-F} = 32.3 Hz), 142.7 (t, CHF₂, J_{C-F} = 29.3 Hz), 121.3 (t, CF₂Cl, J_{C-F} = 287.3 Hz), 110.8 (C_{IV}arom), 109.1 (t, CHF₂, J_{C-F} = 240.2 Hz), 62.0 (CH₂), 13.6 (CH₃) ppm. ¹⁹F NMR (CDCl₃, 282MHz, 25°C): *δ* = -49.6 (CF₂Cl), -116.8 (CHF₂, J_{F-H} = 53.5 Hz) ppm.

### Beispiel 13:

### N-Methyl-3-difluoromethyl-5-pentafluoroethyl-4-pyrazolcarbonsäureethylester:

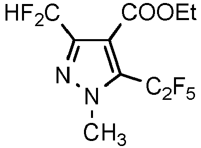

BF₃.OEt₂ (1.24 mL, 10.0 mmol) wurde zu einer Lösung von TFEDMA (1.20 mL, 10.0 mmol) in trockenem Dichlormethan (10 mL) unter Argon in einem Teflonkolben gegeben. Die Lösung wurde 15 min bei Raumtemperatur gerührt, bevor das Dichlormethan unter Vakuum entfernt wurde. Der Rückstand wurde dann in trockenem Acetonitril (10 mL) aufgenommen. In einem zweiten Teflonkolben wurden Ethyl-4,4,5,5,5-pentafluoroacetoacetat (1.75 mL, 10.0 mmol) zu einer Lösung von Pyridin (2.42 mL, 30.0 mmol) in trockenem Acetonitril (20 mL) gegeben und 15 min bei Raumtemperatur gerührt. Hierzu wurde bei -30°C der Inhalt des ersten Kolbens zugetropft. Die Reaktionsmischung wurde im Kältebad auf Raumtemperatur gebracht und über Nacht gerührt. Methylhydrazin (0.79 mL, 15.0 mmol) wurde dann bei Raumtemperatur zugetropft und die Mischung über Nacht gerührt. Das Lösemittel wurde im Vakuum entfernt und der Rückstand durch Flashchromatographie auf Kieselgel mit einer Pentane/Diethylether Mischung (10:0 - 8:2) gereinigt. *N-*Methyl-3-difluoromethyl-5-pentafluoroethyl-4-pyrazolcarbonsäureethylester (2.42 g, 7.52 mmol, 75%) wurde als farblose Flüssigkeit isoliert.

¹H NMR (CDCl₃, 300 MHz, 25 °C): *δ* = 7.00 (t, 1H, CHF₂, J_{H-F} = 53.9 Hz,), 4.35 (q, 2H, CH₂, J = 7.1 Hz), 4.10 (t, 3H, N-CH₃, J_{H-F} = 2.2 Hz), 1.35 (t, 3H, CH₃, J = 7.1 Hz) ppm. ¹³C NMR (CDCl₃, 75 MHz, 25 °C): *δ* = 160.2 (CO), 146.1 (t, C_{IV}arom, J_{C-F} = 25.6 Hz), 131.1 (t, C_{IV}arom, J_{C-F} = 29.6 Hz), 118.6 (qt, CF₂CF₃, J¹_{C-F} = 287.1 Hz, J³_{C-F} = 37.7 Hz), 116.3 (C_{IV}arom), 109.98 (tq, CF₂CF₃, J¹_{C-F} = 192.0 Hz, J³_{C-F} - 41.7 Hz), 109.1 (t, CHF₂, J¹_{C-F} = 238.1 Hz), 61.9 (CH₂), 41.0 (t, N-CH₃, J_{C-F} = 4.3 Hz), 13.8 (CH₃) ppm. ¹⁹F NMR (CDCl₃, 282MHz, 25°C): *δ* = -83.7 (CF₂CF₃), -109.5 (CF₂CF₃), -116.8 (CHF₂, J_{F-H} = 53.9 Hz) ppm.

### Beispiel 14:

### N-Methyl-3-difluoromethyl-5-pentafluoroethyl-4-pyrazolcarbonsäure:

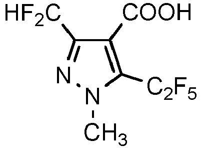

*N*-Methyl-3-difluoromethyl-5-pentafluoroethyl-4-pyrazolcarbonsäureethylester (0.5 g, 1.55 mmol) wurde in Ethanol (3 mL) langsam mit einer 8N wässrigen Natriumhydroxid Lösung (0.6 mL) versetzt und 3h bei Raumtemperatur gerührt. Das Lösemittel wurde abrotiert, der Rückstand in Wasser (10 mL) aufgenommen und mit Diethylether (10 mL) extrahiert. Nach Ansäuern auf pH 1 mit 6M HCl wurde mit Ethylacetat (3 x 10 mL) extrahiert. Die vereinigten organischen Phasen wurden über Natriumsulfat getrocknet, filtriert und das Lösemittel abrotiert. *N*-Methyl-3-difluoromethyl-5-pentafluoroethyl-4-pyrazolcarbonsäure (0.44 g, 1.50 mmol, 97%) wurde als farbloser Feststoff isoliert.

¹H NMR (CDCl₃, 300 MHz, 25 °C): *δ* = 11.16 (brs, 1H, COOH), 7.09 (t, 1H, CHF₂, J_{H-F} = 53.6 Hz,), 4.15 (t, 3H, N-CH₃, J_{H-F} = 2.4 Hz) ppm. ¹³C NMR (CDCl₃, 75 MHz, 25 °C): *δ* = 165.2 (CO), 147.2 (t, C_{IV}arom, J_{C-F} = 25.2 Hz), 132.5 (t, C_{IV}arom, J_{C-F} = 29.8 Hz), 118.5 (qt, CF₂CF₃, J¹_{C-F} = 287.0 Hz, J³_{C-F} = 37.5 Hz), 114.6 (C_{IV}arom), 109.9 (tq, CF₂CF₃, J¹_{C-F} = 258.0 Hz, J³_{C-F} = 41.7 Hz), 108.8 (t, CHF₂, J¹_{C-F} = 238.6 Hz), 41.4 (t, N-CH₃, J_{C-F} = 4.8 Hz) ppm.

### Beispiel 15:

### N-H-3-difluoromethyl-5-pentafluoroethyl-4-pyrazolcarbonsäureethylester:

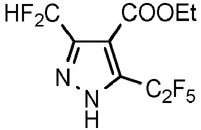

BF₃.OEt₂ (1.24 mL, 10.0 mmol) wurde zu einer Lösung von TFEDMA (1.20 mL, 10.0 mmol) in trockenem Dichlormethan (10 mL) under Argon in einem Teflonkolben gegeben. Die Lösung wurde 15 min bei Raumtemperatur gerührt, bevor das Dichlormethan unter Vakuum entfernt wurde. Der Rückstand wurde dann in trockenem Acetonitril (10 mL) aufgenommen. In einem zweiten Teflonkolben wurde Ethyl-4,4,5,5,5-pentafluoroacetoacetat (1.75 mL, 10.0 mmol) zu einerr Lösung von Pyridin (2.42 mL, 30.0 mmol) in trockenem Acetonitril (20 mL) gegeben und 15 min bei Raumtemperatur gerührt. Hierzu wurde bei -30°C der Inhalt des ersten Kolbens zugetropft. Die Reaktionsmischung wurde im Kältebad auf Raumtemperatur gebracht und über Nacht gerührt. Hydrazinhydrat (0.74 mL, 15.0 mmol) wurde dann bei Raumtemperatur zugetropft und die Mischung über Nacht gerührt. Das Lösemittel wurde im Vakuum entfernt und der Rückstand durch Flashchromatographie auf Kieselgel mit einer Pentane/Diethylether Mischung (10:0 - 8:2) gereinigt. *N*-H-3-difluoromethyl-5-pentafluoroethyl-4-pyrazolcarbonsäureethylester (2.06 g, 6.70 mmol, 67%) wurde als farbloses Öl isoliert.

¹H NMR (CDCl₃, 300 MHz, 25 °C): *δ* = 12.69 (brs, 1H, COOH), 7.26 (t, 1H, CHF₂, J_{H-F} = 53.5 Hz,), 4.40 (q, 2H, CH₂, J = 7.1 Hz), 1.39 (t, 3H, CH₃, J = 7.1 Hz) ppm. ¹³C NMR (CDCl₃, 75 MHz, 25 °C): *δ* = 160.6 (CO), 141.8 (t, C_{IV}arom, J_{C-F} = 25.9 Hz), 141.1 (t, C_{IV}arom, J_{C-F} = 31.7 Hz), 118.7 (qt, CF₂CF₃, J¹_{C-F} = 286.6 Hz, J³_{C-F} = 36.3 Hz), 113.2 (Civarom), 110.1 (tq, CF₂CF₃, J¹_{C-F} = 252.9 Hz, J³_{C-F} = 39.5 Hz), 107.5 (t, CHF₂, J¹_{C-F} = 238.8 Hz), 62.0 (CH₂), 13.6 (CH₃) ppm. ¹⁹F NMR (CDCl₃, 282MHz, 25°C): *δ* = - 83.2 (CF₂CF₃), -110.1 (CF₂CF₃), -117.2 (CHF₂, J_{F-H} = 53.5 Hz) ppm.

### Beispiel 16:

### N-Methyl-3-difluoromethyl-5-trifluoromethyl-4-pyrazolcarbonsäureethylester:

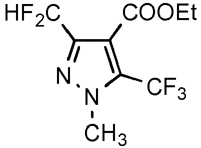

BF₃. (0,34 g , 5 mmol) als 17 % Lösung in Acetonitril (0,76 ml) wurde zu einer Lösung von TFEDMA (0.59 mL, 5.0 mmol) in CH₃CN (5 mL) under Argon in einem Teflonkolben gegeben. Die Lösung wurde 15 min bei Raumtemperatur gerührt. In einem zweiten Teflonkolben wurde Ethyl-4,4,4-trifluoroacetoacetat (0.73 mL, 5.0 mmol) zu einer Lösung von Kaliumfluoride (0.88g, 15.0 mol) in trockenem Acetonitril (10 mL) gegeben und 15 min bei Raumtemperatur gerührt. Hierzu wurde bei - 30°C der Inhalt des ersten Kolbens zugetropft. Die Reaktionsmischung wurde im Kältebad auf Raumtemperatur gebracht und über Nacht gerührt. Methylhydrazin (0.32 mL, 6.0 mmol) wurde dann bei Raumtemperatur zugetropft und die Mischung über Nacht gerührt. Das Lösemittel wurde im Vakuum entfernt und der Rückstand durch Flashchromatographie auf Kieselgel mit einer Pentane/Diethylether Mischung (9:1 - 8:2) gereinigt. *N*-Methyl-3-difluoromethyl-5-trifluoromethyl-4-pyrazolcarbonsäureethylester (0.95 g,) wurde als gelbes Öl erhalten.

### Beispiel 17:

### N-Phenyl-3-difluoromethyl-5-trifluoromethyl-4-pyrazolcarbonsäureethylester:

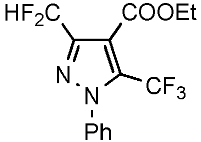

BF₃.OEt₂ (2.5 mL, 20.0 mmol) wurde zu einer Lösung von TFEDMA (2.4 mL, 20.0 mmol) in trockenem Dichlormethan (20 mL) unter Argon in einem Teflonkolben gegeben. Die Lösung wurde 15 min bei Raumtemperatur gerührt, bevor das Dichlormethan unter Vakuum entfernt wurde. Der Rückstand wurde dann in trockenem Acetonitril (20 mL) aufgenommen. In einem zweiten Teflonkolben wurde Ethyl-4,4,4-trifluoroacetoacetat (2.8 mL, 20.0 mmol) zu einer Lösung von Pyridin (4.7 g, 60.0 mmol) in trockenem Acetonitril (40 mL) gegeben und 15 min bei Raumtemperatur gerührt. Hierzu wurde bei -30°C der Inhalt des ersten Kolbens zugetropft. Die Reaktionsmischung wurde im Kältebad auf Raumtemperatur gebracht und über Nacht gerührt. Phenylhydrazin (3.0 mL, 30.0 mmol) wurde dann bei Raumtemperatur zugetropft und die Mischung über Nacht gerührt. Das Lösemittel wurde im Vakuum entfernt und der Rückstand durch Flashchromatographie auf Kieselgel mit einer Pentane/Diethylether Mischung (9:1) gereinigt. *N*-Phenyl-3-difluoromethyl-5-trifluoromethyl-4-pyrazolcarbonsäureethylester (4.47 g, 13.4 mmol, 67%) wurde als farbloser Feststoff isoliert.

¹H NMR (CDCl₃, 300 MHz, 25 °C): *δ* = 7.55-7.42 (m, 5H, N-Ph), 7.05 (t, 1H, CHF₂, J_{H-F} = 53.7 Hz), 4.42 (q, 2H, CH₂, J = 7.1 Hz), 1.40 (t, 3H, CH₃, J = 7.1 Hz) ppm. ¹³C NMR (CDCl₃, 75 MHz, 25 °C): *δ* = 160.3 (CO), 146.7 (t, C_{IV}arom, J_{C-F} = 26.2 Hz), 138.8 (N-C_{IV} Phenyl), 133.8 (q, Civarom, J_{C-F} = 40.1 Hz), 130.4 (CH Phenyl), 129.3 (CH Phenyl), 125.9 (CH Phenyl), 118.6 (q, CF₃, J_{C-F} = 271.9 Hz), 115.0 (C_{IV}arom), 109.2 (t, CHF₂, J_{C-F} = 238.4 Hz), 62.0 (CH₂), 13.8 (CH₃) ppm. ¹⁹F NMR (CDCl₃, 282MHz, 25°C): *δ* = -56.8 (CF₃), -117.3 ppm.

### Beispiel 18:

### N-Phenyl-3-difluoromethyl-5-trifluoromethyl-4-pyrazolcarbonsäure:

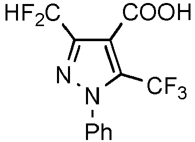

*N*-Phenyl-3-difluoromethyl-5-trifluoromethyl-4-pyrazolcarbonsäureethylester (3.0 g, 9.0 mmol) wurde in Ethanol (15 mL) langsam mit einer 8N wässrigen Natriumhydroxid Lösung (3.4 mL) versetzt und 3h bei Raumtemperatur gerührt. Das Lösemittel wurde abrotiert, der Rückstand in Wasser (40 mL) aufgenommen und mit Diethylether (20 mL) extrahiert. Nach Ansäuern auf pH 1 mit 6M HCl wurde mit Ethylacetat (3 x 30 mL) extrahiert. Die vereinigten organischen Phasen wurden über Natriumsulfat getrocknet, filtriert und das Lösemittel abrotiert. *N*-Phenyl-3-difluoromethyl-5-trifluoromethyl-4-pyrazolcarbonsäure (2.58 g, 8.43 mmol, 94%) wurde als farbloser Feststoff isoliert.

¹H NMR (CDCl₃, 300 MHz, 25 °C): *δ* = 11.53 (brs, 1H, -COOH), 7.58-7.44 (m, 5H, N-Phenyl), 7.15 (t, 1H, CHF₂, J_{H-F} = 53.5 Hz) ppm. ¹³C NMR (CDCl₃, 75 MHz, 25 °C): *δ* = 165.8 (CO), 147.6 (t, C_{IV}arom, J_{C-F} = 25.8 Hz), 138.7 (N-C_{IV} Phenyl), 135.1 (q, C_{IV}arom, J_{C-F} = 40.4 Hz), 130.6 (CH Phenyl), 129.4 (CH Phenyl), 125.9 (CH Phenyl), 118.4 (q, CF₃, J_{C-F} = 272.3 Hz), 114.3 (C_{IV}arom), 108.9 (t, CHF₂, J_{C-F} = 239.0 Hz) ppm. ¹⁹F NMR (CDCl₃, 282MHz, 25°C): *δ* = -56.8 (CF₃), -117.8 (CHF₂) ppm.

### Beispiel 19:

### N-Phenyl-3-difluoromethyl-5-chlorodifluoromethyl-4-pyrazolcarbonsäureethylester:

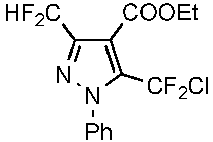

BF₃.OEt₂ (2.5 mL, 20.0 mmol) wurde zu einer Lösung von TFEDMA (2.4 mL, 20.0 mmol) in trockenem Dichlormethan (20 mL) unter Argon in einem Teflonkolben gegeben. Die Lösung wurde 15 min bei Raumtemperatur gerührt, bevor das Dichlormethan unter Vakuum entfernt wurde. Der Rückstand wurde dann in trockenem Acetonitril (20 mL) aufgenommen. In einem zweiten Teflonkolben wurde Ethyl-4-chloro-4,4-difluoroacetoacetat (4.0 g, 20.0 mmol) zu einer Lösung von Pyridin (4.7 g, 60.0 mmol) in trockenem Acetonitril (40 mL) gegeben und 15 min bei Raumtemperatur gerührt. Hierzu wurde bei -30°C der Inhalt des ersten Kolbens zugetropft. Die Reaktionsmischung wurde im Kältebad auf Raumtemperatur gebracht und über Nacht gerührt. Phenylhydrazin (3.0 mL, 30.0 mmol) wurde dann bei Raumtemperatur zugetropft und die Mischung über Nacht gerührt. Das Lösemittel wurde im Vakuum entfernt und der Rückstand durch Flashchromatographie auf Kieselgel mit einer Pentane/Diethylether Mischung (9:1) gereinigt. *N*-Phenyl-3-difluoromethyl-5-chlorodifluoromethyl-4-pyrazolcarbonsäureethylester (3.67 g, 10.5 mmol, 53%) wurde als farbloser Feststoff isoliert.

¹H NMR (CDCl₃, 300 MHz, 25 °C): *δ* = 7.55-7.45 (m, 5H, N-Ph), 7.03 (t, 1H, CHF₂, J_{H-F} = 53.7 Hz), 4.42 (q, 2H, CH₂, J = 7.1 Hz), 1.41 (t, 3H, CH₃, J = 7.2 Hz) ppm. ¹³C NMR (CDCl₃, 75 MHz, 25 °C): *δ* = 160.5 (CO), 146.5 (t, C_{IV}arom, J_{C-F} = 26.3 Hz), 138.9 (N-C_{IV} Phenyl), 138.3 (t, C_{IV}arom, J_{C-F} = 32.7 Hz), 130.3 (CH Phenyl), 129.2 (CH Phenyl), 126.2 (CH Phenyl), 119.5 (t, CF₃, J_{C-F} = 290.0 Hz), 115.6 (C_{IV}arom), 109.3 (t, CHF₂, J_{C-F} = 238.4 Hz), 62.0 (CH₂), 13.9 (CH₃) ppm. ¹⁹F NMR (CDCl₃, 282MHz, 25°C): *δ* = -46.6 (CF₂Cl), -117.3 (CHF₂) ppm.

### Beispiel 20:

### N-Phenyl-3-difluoromethyl-5-chlorodifluoromethyl-4-pyrazolcarbonsäure:

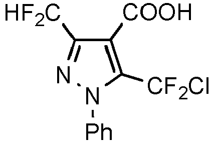

*N*-Phenyl-3-difluoromethyl-5-chlorodifluoromethyl-4-pyrazolcarbonsäureethylester (3.0 g, 8.56 mmol) wurden in Ethanol (15 mL) langsam mit einer 8N wässrigen Natriumhydroxid Lösung (3.2 mL) versetzt und 3h bei Raumtemperatur gerührt. Das Lösemittel wurde abrotiert, der Rückstand in Wasser (40 mL) aufgenommen und mit Diethylether (20 mL) extrahiert. Nach Ansäuern auf pH 1 mit 6M HCl wurde mit Ethylacetat (3 x 30 mL) extrahiert. Die vereinigten organischen Phasen wurden über Natriumsulfat getrocknet, filtriert und das Lösemittel abrotiert. *N-*Phenyl-3-difluoromethyl-5-chlorodifluoromethyl-4-pyrazolcarbonsäure (2.74 g, 8.49 mmol, 99%) wurde als farbloser Feststoff isoliert.

¹H NMR (CDCl₃, 300 MHz, 25 °C): *δ* = 7.57-7.47 (m, 5H, N-Phenyl), 7.12 (t, 1H, CHF₂, J_{H-F} = 53.5 Hz) ppm. ¹³C NMR (CDCl₃, 75 MHz, 25 °C): *δ* = 165.9 (CO), 147.4 (t, C_{IV}arom, J_{C-F} = 25.8 Hz), 139.8 (t, C_{IV}arom, J_{C-F} = 33.0 Hz), 138.9 (N-C_{IV} Phenyl), 130.5 (CH Phenyl), 129.3 (CH Phenyl), 126.2 (CH Phenyl), 119.2 (t, CF₂Cl, J_{C-F} = 290.6 Hz), 112.1 (C_{IV}arom), 108.9 (t, CHF₂, J_{C-F} = 239.0 Hz) ppm. ¹⁹F NMR (CDCl₃, 282MHz, 25°C): *δ* = -46.9 (CF₂Cl), -117.8 (CHF₂) ppm.

### Beispiel 21:

### N-Phenyl-3-difluoromethyl-5-pentafluoroethyl-4-pyrazolcarbonsäureethylester:

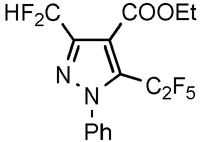

BF₃.OEt₂ (2.5 mL, 20.0 mmol) wurde zu einer Lösung von TFEDMA (2.4 mL, 20.0 mmol) in trockenem Dichlormethan (20 mL) unter Argon in einem Teflonkolben gegeben. Die Lösung wurde 15 min bei Raumtemperatur gerührt, bevor das Dichlormethan unter Vakuum entfernt wurde. Der Rückstand wurde dann in trockenem Acetonitril (20 mL) aufgenommen. In einem zweiten Teflonkolben wurde Ethyl-4,4,5,5,5-pentafluoroacetoacetat (3.5 mL, 11.4 mmol) zu einer Lösung von Pyridin (2.7 g, 34.4 mmol) in trockenem Acetonitril (40 mL) gegeben und 15 min bei Raumtemperatur gerührt. Hierzu wurde bei -30°C der Inhalt des ersten Kolbens zugetropft. Die Reaktionsmischung wurde im Kältebad auf Raumtemperatur gebracht und über Nacht gerührt. Phenylhydrazin (2.0 mL, 20.0 mmol) wurde dann bei Raumtemperatur zugetropft und die Mischung über Nacht gerührt. Das Lösemittel wurde im Vakuum entfernt und der Rückstand durch Flashchromatographie auf Kieselgel mit einer Pentane/Diethylether Mischung (9:1) gereinigt. *N-*Phenyl-3-difluoromethyl-5-pentafluoroethyl-4-pyrazolcarbonsäureethylester (3.73 g, 9.70 mmol, 85%) wurde als beiger Feststoff isoliert.

¹H NMR (CDCl₃, 300 MHz, 25 °C): *δ* = 7.58-7.35 (m, 5H, N-Ph), 7.04 (t, 1H, CHF₂, J_{H-F} = 53.8 Hz), 4.40 (q, 2H, CH₂, J = 7.1 Hz), 1.38 (t, 3H, CH₃, J = 7.2 Hz) ppm. ¹³C NMR (CDCl₃, 75 MHz, 25 °C): *δ* = 165.8 (CO), 147.6 (t, C_{IV}arom, J_{C-F} = 25.8 Hz), 138.7 (N-C_{IV} Phenyl), 135.1 (q, C_{IV}arom, J_{C-F} = 40.4 Hz), 130.6 (CH Phenyl), 129.4 (CH Phenyl), 125.9 (CH Phenyl), 118.4 (qt, CF₃, J¹_{C-F} = 287.5 Hz, J³_{C-F} = 37.5 Hz), 116.4 (C_{IV}arom), 109.6 (tq, CF₂, J¹_{C-F} = 255.3 Hz, J³C-_{F} = 41.6 Hz), 109.4 (t, CHF₂, J_{C-F} = 238.6 Hz), 62.1 (CH₂), 13.7 (CH₃) ppm. ¹⁹F NMR (CDCl₃, 282MHz, 25°C): *δ* = -83.6 (CF₃), -107.1 (CF₂), -117.3 (CHF₂) ppm.

### Beispiel 22:

### N-Phenyl-3-difluoromethyl-5-pentafluoroethyl-4-pyrazolcarbonsäure:

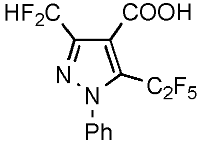

*N*-Phenyl-3-difluoromethyl-5-pentafluoroethyl-4-pyrazolcarbonsäureethylester (3.0 g, 7.81 mmol) wurde in Ethanol (15 mL) langsam mit einer 8N wässrigen Natriumhydroxid Lösung (3.0 mL) versetzt und 3h bei Raumtemperatur gerührt. Das Lösemittel wurde abrotiert, der Rückstand in Wasser (40 mL) aufgenommen und mit Diethylether (20 mL) extrahiert. Nach Ansäuern auf pH 1 mit 6M HCl wurde mit Ethylacetat (3 x 30 mL) extrahiert. Die vereinigten organischen Phasen wurden über Natriumsulfat getrocknet, filtriert und das Lösemittel abrotiert. *N*-Phenyl-3-difluoromethyl-5-pentafluoroethyl-4-pyrazolcarbonsäure (2.71 g, 7.61 mmol, 98%) wurde als farbloser Feststoff isoliert.

¹H NMR (CDCl₃, 300 MHz, 25 °C): *δ* = 7.60-7.37 (m, 5H, N-Phenyl), 7.14 (t, 1H, CHF₂, J_{H-F}= 53.6 Hz) ppm. ¹³C NMR (MeOD, 75 MHz, 25 °C): *δ* = 164.0 (CO), 148.6 (t, C_{IV}arom, J_{C-F} = 25.6 Hz), 141.4 (N-C_{IV} Phenyl), 133.4 (CH Phenyl), 133.1 (t, C_{IV}arom, J_{C-F} = 29.1 Hz), 131.7 (CH Phenyl), 130.0 (CH Phenyl), 120.6 (qt, CF₃, J¹_{C-F} = 287.6 Hz, J³_{C-F} = 37.9 Hz), 120.1 (C_{IV}arom), 112.3 (t, CHF₂, J_{C-F} = 236.4Hz), 112.1 (tq, CF₂, J¹_{C-F} = 262.5 Hz, J³_{C-F} = 40.5 Hz) ppm. ¹⁹F NMR (CDCl₃, 282MHz, 25°C): *δ* = -83.5 (CF₃), -107.1 (CF₂), -117.9 (CHF₂) ppm.

### Beispiel 23:

### N-tert-Butyl-3-difluoromethyl-5-trifluoromethyl-4-pyrazolcarbonsäureethylester:

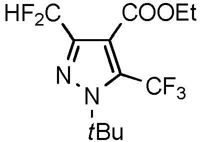

BF₃.OEt₂ (2.7 mL, 22.0 mmol) wurde zu einer Lösung von TFEDMA (2.5 mL, 22.0 mmol) in trockenem Dichlormethan (20 mL) unter Argon in einem Teflonkolben gegeben. Die Lösung wurde 15 min bei Raumtemperatur gerührt, bevor das Dichlormethan unter Vakuum entfernt wurde. Der Rückstand wurde dann in trockenem Acetonitril (20 mL) aufgenommen. In einem zweiten Teflonkolben wurde Ethyl-4,4,4-trifluoroacetoacetat (2.8 mL, 20.0 mmol) zu einer Lösung von Pyridin (7.1 g, 90.0 mmol) in trockenem Acetonitril (40 mL) gegeben und 15 min bei Raumtemperatur gerührt. Hierzu wurde bei -30°C der Inhalt des ersten Kolbens zugetropft. Die Reaktionsmischung wurde im Kältebad auf Raumtemperatur gebracht und über Nacht gerührt. *tert*-Butyl hydrazin hydrochlorid (3.74 g, 30.0 mmol) wurde zu eine Lösung von Kaliumhydroxid (1.68 g, 30mmol) in methanol (10 mL) gegeben und 30 Minuten bei Raumtemperatur gerührt. Diese Mischung wurde dann zur vorher hergestellten Zwischenverbindung (Ethyl 2-(2,2,2-trifluoroacetyl)-3-(dimethylamino)-4,4-difluorobut-2-enoate) hinzugegeben und über Nacht gerührt. Das Lösemittel wurde im Vakuum entfernt und der Rückstand durch Flashchromatographie auf Kieselgel mit einer Pentane/Diethylether Mischung (9:1) gereinigt. *N-tert*-Butyl-3-difluoromethyl-5-trifluoromethyl-4-pyrazolcarbonsäureethylester (3.29 g, 10.5 mmol, 53%) wurde als gelbes Öl isoliert.

¹H NMR (CDCl₃, 300 MHz, 25 °C): *δ* = 6.80 (t, 1H, CHF₂, J_{H-F} = 54.0 Hz), 4.37 (q, 2H, CH₂, J = 7.1 Hz), 1.70 (s, 9H, *t*Bu), 1.36 (t, 3H, CH₃, J = 7.1 Hz) ppm. ¹³C NMR (CDCl₃, 75 MHz, 25 °C): *δ* = 161.5 (CO), 141.9 (t, C_{IV}arom, J_{C-F} = 27.8 Hz), 131.5 (q, C_{IV}arom, J_{C-F} = 40.6 Hz), 119.3 (q, CF₃, J_{C-F} = 270.7 Hz), 116.9 (C_{IV}arom), 109.9 (t, CHF₂, J_{C-F} = 236.7 Hz), 66.0 (N-C_{IV} *t*Bu), 62.0 (CH₂), 29.9 (q, CH₃ *t*Bu, J_{C-F} = 2.4 Hz), 13.8 (CH₃) ppm. ¹⁹F NMR (CDCl₃, 282MHz, 25°C): *δ* = -53.3 (CF₃), -114.4 (CHF₂, J_{F-H} = 54.0 Hz) ppm.

### Beispiel 24:

### N-tert-Butyl-3-difluoromethyl-5-trifluoromethyl-4-pyrazolcarbonsäure:

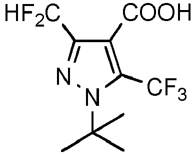

*N-tert*-Butyl-3-difluoromethyl-5-trifluoromethyl-4-pyrazolcarbonsäureethylester (2.48 g, 7.9 mmol) wurde in Ethanol (15 mL) langsam mit einer 8N wässrigen Natriumhydroxid Lösung (3.0 mL) versetzt und 3h bei Raumtemperatur gerührt. Das Lösemittel wurde abrotiert, der Rückstand in Wasser (40 mL) aufgenommen und mit Diethylether (20 mL) extrahiert. Nach Ansäuern auf pH 1 mit 6M HCl wurde mit Ethylacetat (3x30 mL) extrahiert. Die vereinigten organischen Phasen wurden über Natriumsulfat getrocknet, filtriert und das Lösemittel abrotiert. *N-tert*-Butyl-3-difluoromethyl-5-trifluoromethyl-4-pyrazolcarbonsäure (2.15 g, 7.52 mmol, 94%) wurde als gelber Feststoff isoliert.

¹H NMR (CDCl₃, 300 MHz, 25 °C): *δ* = 6.92 (t, 1H, CHF₂, J_{H-F} = 53.8 Hz), 1.74 (s, 9H, *t*Bu) ppm. ¹³C NMR (CDCl₃, 75 MHz, 25 °C): *δ* = 166.8 (CO), 142.9 (t, C_{IV}arom, J_{C-F} = 26.9 Hz), 132.9 (q, C_{IV}arom, J_{C-F} = 41.1 Hz), 119.1 (q, CF₃, J_{C-F} = 271.1 Hz), 115.1 (C_{IV}arom), 109.5 (t, CHF₂, J_{C-F} = 237.5 Hz), 66.7 (N-C_{IV} *t*Bu), 29.9 (q, CH₃ *t*Bu, J_{C-F} = 2.5 Hz) ppm. ¹⁹F NMR (CDCl₃, 282MHz, 25°C): *δ* = -54.0 (CF₃), - 116.0 (CHF₂) ppm.

### Beispiel 25:

### N-tert-Butyl-3-difluoromethyl-5-pentafluoroethyl-4-pyrazolcarbonsäureethylester:

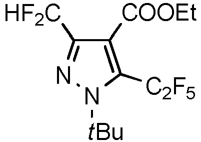

BF₃.OEt₂ (2.7 mL, 22.0 mmol) wurde zu einer Lösung von TFEDMA (2.5 mL, 22.0 mmol) in trockenem Dichlormethan (20 mL) unter Argon in einem Teflonkolben gegeben. Die Lösung wurde 15 min bei Raumtemperatur gerührt, bevor das Dichlormethan unter Vakuum entfernt wurde. Der Rückstand wurde dann in trockenem Acetonitril (20 mL) aufgenommen. In einem zweiten Teflonkolben wurde Ethyl-4,4,5,5,5-pentafluoroacetoacetat (4.68 g, 20.0 mmol) zu einer Lösung von Pyridin (7.1 g, 90.0 mmol) in trockenem Acetonitril (40 mL) gegeben und 15 min bei Raumtemperatur gerührt. Hierzu wurde bei -30°C der Inhalt des ersten Kolbens zugetropft. Die Reaktionsmischung wurde im Kältebad auf Raumtemperatur gebracht und über Nacht gerührt. *tert*-Butyl hydrazin hydrochlorid (3.74 g, 30.0 mmol) wurde dann bei Raumtemperatur zugetropft und die Mischung über Nacht gerührt. Das Lösemittel wurde im Vakuum entfernt und der Rückstand durch Flashchromatographie auf Kieselgel mit einer Pentane/Diethylether Mischung (9:1) gereinigt. *N-tert*-Butyl-3-difluoromethyl-5-pentafluoroethyl-4-pyrazolcarbonsäureethylester (2.41 g, 6.61 mmol, 33%) wurde als farbloses Öl isoliert.

¹H NMR (CDCl₃, 300 MHz, 25 °C): *δ* = 6.83 (t, 1H, CHF₂, J_{H-F} = 54.1 Hz), 4.35 (q, 2H, CH₂, J = 7.1 Hz), 1.69 (s, 9H, *t*Bu), 1.34 (t, 3H, CH₃, J = 7.2 Hz) ppm. ¹³C NMR (CDCl₃, 75 MHz, 25 °C):*δ*= 161.2 (CO), 142.8 (t, C_{IV}arom, J_{C-F} = 27.3 Hz), 130.0 (q, C_{IV}arom, J_{C-F} = 31.0 Hz), 118.6 (qt, CF₃, J¹_{C-F} = 287.8 Hz, J³_{C-F} = 38.3 Hz), 118.5 (C_{IV}arom), 110.8 (tq, CF₂, J¹_{C-F} = 258.1 Hz, J³C-F = 41.0 Hz), 110.0 (t, CHF₂, J_{C-F} = 237.2 Hz), 67.6 (N-C_{IV} *t*Bu), 62.0 (CH₂), 30.5 (t, CH₃ *t*Bu, J_{C-F} = 3.6 Hz), 13.7 (CH₃) ppm. ¹⁹F NMR (CDCl₃, 282MHz, 25°C): *δ* = -80.7 (CF₃), -100.8 (CF₂), -115.5 (CHF₂, J_{F-H} = 54.1 Hz) ppm.

### Beispiel 26:

### N-tert-Butyl-3-difluoromethyl-5-pentafluoroethyl-4-pyrazolcarbonsäure:

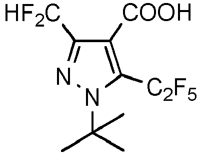

*N-tert*-Butyl-3-difluoromethyl-5-pentafluoroethyl-4-pyrazolcarbonsäureethylester (2.0 g, 5.50 mmol) wurde in Ethanol (10 mL) langsam mit einer 8N wässrigen Natriumhydroxid Lösung (2.0 mL) versetzt und 3h bei Raumtemperatur gerührt. Das Lösemittel wurde abrotiert, der Rückstand in Wasser (40 mL) aufgenommen und mit Diethylether (20 mL) extrahiert. Nach Ansäuern auf pH 1 mit 6M HCl wurde mit Ethylacetat (3 x 30 mL) extrahiert. Die vereinigten organischen Phasen wurden über Natriumsulfat getrocknet, filtriert und das Lösemittel abrotiert. *N-tert*-Butyl-3-difluoromethyl-5-pentafluoroethyl-4-pyrazolcarbonsäure (1.83 g, 5.44 mmol, 99%) wurde als gelber Feststoff isoliert.

¹H NMR (CDCl₃, 300 MHz, 25 °C): *δ* = 11.4 (brs, 1H, COOH), 7.01 (t, 1H, CHF₂, J_{H-F} = 53.9 Hz), 1.78 (s, 9H, *t*Bu) ppm. ¹³C NMR (CDCl₃, 75 MHz, 25 °C): *δ* = 166.5 (CO), 143.9 (t, C_{IV}arom, J_{C-F} = 26.3 Hz), 131.5 (q, C_{IV}arom, J_{C-F} = 31.0 Hz), 120.0 (qt, CF₃, J¹_{C-F} = 288.1 Hz, J³_{C-F} = 38.1 Hz), 117.4 (C_{IV}arom), 110.6 (tq, CF₂, J¹_{C-F} = 258.7 Hz, J³_{C-F} = 41.2 Hz), 109.5 (t, CHF₂, J_{C-F} = 237.9 Hz), 68.3 (N-C_{IV} *t*Bu), 30.6 (t, CH₃ *t*Bu, J_{C-F} = 3.7 Hz) ppm. ¹⁹F NMR (CDCl₃, 282MHz, 25°C): *δ* = -80.3 (CF₃), -100.4 (CF₂), - 116.3 (CHF₂, J_{F-H} = 53.9 Hz) ppm.

### Beispiel 27:

### N-tert-Butyl-3,5-bis(difluoromethyl)-4-pyrazolcarbonsäureethylester:

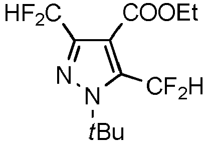

BF₃.OEt₂ (2.7 mL, 22.0 mmol) wurde zu einer Lösung von TFEDMA (2.5 mL, 22.0 mmol) in trockenem Dichlormethan (20 mL) unter Argon in einem Teflonkolben gegeben. Die Lösung wurde 15 min bei Raumtemperatur gerührt, bevor das Dichlormethan unter Vakuum entfernt wurde. Der Rückstand wurde dann in trockenem Acetonitril (20 mL) aufgenommen. In einem zweiten Teflonkolben wurde Ethyl-4,4-difluoroacetoacetat (2.8 mL, 20.0 mmol) zu einer Lösung von Pyridin (7.1 g, 90.0 mmol) in trockenem Acetonitril (40 mL) gegeben und 15 min bei Raumtemperatur gerührt. Hierzu wurde bei -30°C der Inhalt des ersten Kolbens zugetropft. Die Reaktionsmischung wurde im Kältebad auf Raumtemperatur gebracht und über Nacht gerührt. *tert*-butyl hydrazin hydrochlorid (3.74 g, 30.0 mmol) wurde zu eine Lösung von Kaliumhydroxid (1.68 g, 30mmol) in methanol (10 mL) gegeben und 30 Minuten bei Raumtemperatur gerührt. Diese Mischung wurde dann zur vorher hergestellten Zwischenverbindung (Ethyl 2-(2,2-difluoroacetyl)-3-(dimethylamino)-4,4-difluorobut-2-enoate) hinzugegeben und über Nacht gerührt. Das Lösemittel wurde im Vakuum entfernt und der Rückstand durch Flashchromatographie auf Kieselgel mit einer Pentane/Diethylether Mischung (9:1) gereinigt. *N-tert*-Butyl-3,5-di(difluoromethyl)- 4-pyrazolcarbonsäureethylester (1.77 g, 5.98 mmol, 30%) wurde als oranges Öl isoliert.

¹H NMR (CDCl₃, 300 MHz, 25 °C): *δ* = 7.71 (t, 1H, CHF₂, J_{H-F} = 52.9 Hz), 6.97 (t, 1H, CHF₂, J_{H-F} = 54.0 Hz), 4.37 (q, 2H, CH₂, J = 7.1 Hz), 1.71 (s, 9H, *t*Bu), 1.39 (t, 3H, CH₃, J = 7.1 Hz) ppm. ¹³C NMR (CDCl₃, 75 MHz, 25 °C): *δ*= 161.9 (CO), 143.4 (t, C_{IV}arom, J_{C-F} = 25.5 Hz), 137.9 (t, C_{IV}arom, J_{C-F} = 24.8 Hz), 114.5 (C_{IV}arom), 109.9 (t, CHF₂, J_{C-F} = 237.3 Hz), 106.8 (t, CHF₂, J_{C-F} = 238.3 Hz), 65.3 (N-C_{IV} *t*Bu), 61.5 (CH₂), 30.0 (t, CH₃ *t*Bu, J_{C-F} = 3.4 Hz), 14.0 (CH₃) ppm. ¹⁹F NMR (CDCl₃, 282MHz, 25°C): *δ* = -111.5 (CHF₂), -116.0 (CHF₂) ppm.

### Beispiel 28:

### N-tert-Butyl-3,5-bis(difluoromethyl)-4-pyrazolcarbonsäure:

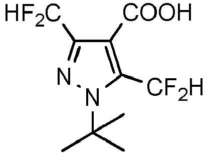

*N-tert*-Butyl-3,5-di(difluoromethyl)-4-pyrazolcarbonsäureethylester (3.40 g, 11.5 mmol) wurde in Ethanol (23 mL) langsam mit einer 8N wässrigen Natriumhydroxid Lösung (4.3 mL) versetzt und 3h bei Raumtemperatur gerührt. Das Lösemittel wurde abrotiert, der Rückstand in Wasser (40 mL) aufgenommen und mit Diethylether (20 mL) extrahiert. Nach Ansäuern auf pH 1 mit 6M HCl wurde mit Ethylacetat (3 x 30 mL) extrahiert. Die vereinigten organischen Phasen wurden über Natriumsulfat getrocknet, filtriert und das Lösemittel abrotiert. *N-tert*-butyl-3,5-di(difluoromethyl)-4-pyrazolcarbonsäure (3.0 g, 11.2 mmol, 97%) wurde als leicht rötlicher Feststoff isoliert.

¹H NMR (CDCl₃, 300 MHz, 25 °C): *δ* = 7.72 (t, 1H, CHF₂, J_{H-F} = 52.7 Hz), 7.06 (t, 1H, CHF₂, J_{H-F} = 53.7 Hz), 1.75 (s, 9H, *t*Bu) ppm. ¹³C NMR (CDCl₃, 75 MHz, 25 °C): *δ* = 167.25 (CO), 144.5 (t, C_{IV}arom, J_{C-F} = 25.3 Hz), 138.8 (q, C_{IV}arom, J_{C-F} = 25.1 Hz), 113.0 (C_{IV}arom), 109.4 (t, CF₂H, J_{C-F} = 237.7 Hz), 106.5 (t, CHF₂, J_{C-F} = 238.8 Hz), 65.9 (N-C_{IV} *t*Bu), 30.0 (t, CH₃ *t*Bu, J_{C-F} = 3.5 Hz) ppm. ¹⁹F NMR (CDCl₃, 282MHz, 25°C): *δ* = -112.5 (CHF₂), -117.4 (CHF₂) ppm.

## Patentansprüche

1. Verfahren zur Herstellung von 3,5-Bis(fluoralkyl)-pyrazolen der Formel (Ia) sowie (Ib), 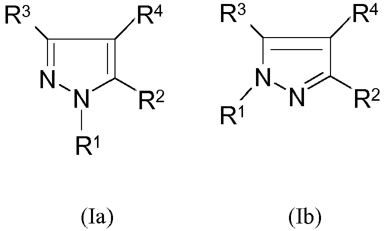 in welcher
R¹ ausgewählt ist aus der Gruppe enthaltend H, C₁₋₁₂-Alkyl, C₃₋₈-Cycloalkyl, C₆₋₁₈-Aryl, C₇₋₁₉-Arylalkyl oder C₇₋₁₉-Alkylaryl, CH₂CN, CH₂CX₃, CH₂COOH, CH₂COO-(C₁₋₁₂)-Alkyl, und
X steht unabhängig voneinander für F, Cl, Br, I;
R² und R³ unabhängig voneinander ausgewählt ist aus C₁-C₆-Halogenalkylgruppen;
R⁴ ausgewählt ist aus der Gruppe enthaltend Hal, COOH, (C=O)OR⁵, CN und (C=O)NR⁵R⁶, wobei R⁵ und R⁶ unabhängig voneinander ausgewählt sind aus der Gruppe enthaltend C₁₋₁₂-Alkyl, C₃₋₈-Cycloalkyl, C₆₋₁₈-Aryl-, C₇₋₁₉-Arylalkyl oder C₇₋₁₉-Alkylaryl oder wobei R⁵ und R⁶ gemeinsam mit dem N-Atom an das sie geknüpft sind einen fünf oder sechsgliedrigen Ring bilden können;
**dadurch gekennzeichnet, dass** in Schritt A) die Umsetzung von α, α -Dihalogenaminen der Formel (II) 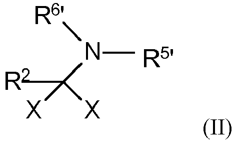 in welcher X für Cl oder F steht, und R⁵' und R⁶' unabhängig voneinander ausgewählt sind aus der Gruppe enthaltend C₁₋₆-Alkyl,
mit Verbindungen der Formel (III), 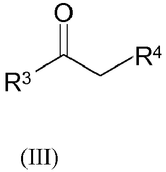 in welcher die Reste R² und R³ die oben angegebene Bedeutung haben, erfolgt und in Schritt B) die Umsetzung mit Hydrazinen der Formel (IV) 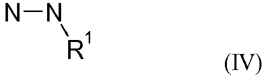 in welcher R¹ die oben angegebene Bedeutung hat, erfolgt.

2. Verfahren gemäß Anspruch 1, **dadurch gekennzeichnet, dass**
R¹ ausgewählt ist aus der Gruppe enthaltend H, C₁₋₁₂-Alkyl, CH₂CN, CH₂COO-(C₁₋₁₂)-Alkyl, und
R² und R³ unabhängig voneinander ausgewählt sind aus der Gruppe enthaltend CF₃, CF₂H, CF₂Cl;
R⁴ ausgewählt ist aus der Gruppe enthaltend COOH, (C=O)OR⁵, CN und (C=O)NR⁵R⁶, wobei R⁵ und R⁶ unabhängig voneinander ausgewählt sind aus der Gruppe enthaltend C₁₋₁₂-Alkyl, C₃₋₈-Cycloalkyl, C₆₋₁₈-Aryl-, C₇₋₁₉-Arylalkyl oder C₇₋₁₉-Alkylaryl oder wobei R⁵und R⁶ gemeinsam mit dem N-Atom an das sie geknüpft sind einen fünf oder sechsgliedrigen Ring bilden können.

3. Verfahren gemäß Anspruch 1, **dadurch gekennzeichnet, dass**
R¹ ausgewählt ist aus der Gruppe enthaltend H, CH₃, CH₂COO-(C₁₋₁₂)-Alkyl, und
R² und R³ unabhängig voneinander ausgewählt sind aus der Gruppe enthaltend CF₃, CF₂H, CF₂Cl;
R⁴ ausgewählt ist aus der Gruppe enthaltend COOH, (C=O)OR⁵.

4. Verbindung der Formel (VI) 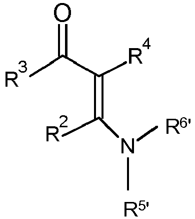 in welcher
R² und R³ unabhängig voneinander ausgewählt sind aus der Gruppe enthaltend CF₃, CF₂H, CF₂Cl;
R⁴ ausgewählt ist aus der Gruppe enthaltend (C=O)OR⁵;
R⁵' und R⁶' unabhängig voneinander ausgewählt sind aus der Gruppe enthaltend C₁₋₆-Alkyl.

## Claims

1. Process for preparing 3,5-bis(fluoroalkyl)pyrazoles of the formula (Ia) and (Ib) 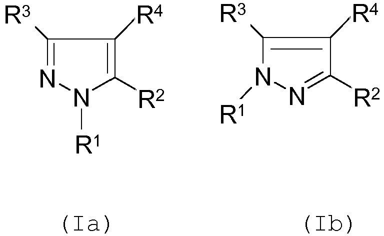 in which
R¹ is selected from the group comprising H, C₁₋₁₂-alkyl, C₃₋₈-cycloalkyl, C₆₋₁₈-aryl, C₇₋₁₉-arylalkyl or C₇₋₁₉-alkylaryl, CH₂CN, CH₂CX₃, CH₂COOH, CH₂COO-(C₁-₁₂)-alkyl, and
X is independently F, Cl, Br, I;
R² and R³ is each independently selected from C₁-C₆-haloalkyl groups;
R⁴ is selected from the group comprising, Hal, COOH, (C=O)OR⁵, CN and (C=O)NR⁵R⁶, where R⁵ and R⁶ are each independently selected from the group comprising C₁₋₁₂-alkyl, C₃₋₈-cycloalkyl, C₆₋₁₈-aryl, C₇₋₁₉-arylalkyl and C₇₋₁₉-alkylaryl, or where R⁵ and R⁶ together with the nitrogen atom to which they are bonded may form a five- or six-membered ring;
**characterized in that**, in step A), α,α-dihaloamines of the formula (II) 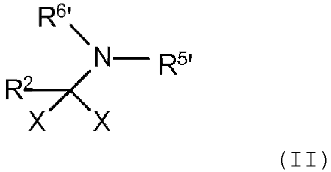 in which X is Cl or F, and R⁵' and R⁶' are each independently selected from the group comprising C₁₋₆-alkyl, are reacted with compounds of the formula (III) 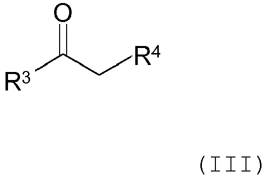 in which the R² and R³ radicals are each as defined above and, in step B), the product is reacted with hydrazines of the formula (IV) 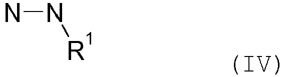 in which R¹ is as defined above.

2. Process according to Claim 1, **characterized in that**
R¹ is selected from the group comprising H, C₁₋₁₂-alkyl, CH₂CN, CH₂COO-(C₁₋₁₂)-alkyl, and
R² and R³ are each independently selected from the group comprising CF₃, CF₂H, CF₂Cl;
R⁴ is selected from the group comprising COOH, (C=O)OR⁵, CN and (C=O)NR⁵R⁶, where R⁵ and R⁶ are each independently selected from the group comprising C₁₋₁₂-alkyl, C₃₋₈-cycloalkyl, C₆₋₁₈-aryl, C₇₋₁₉-arylalkyl and C₇₋₁₉-alkylaryl, or where R⁵ and
R⁶ together with the nitrogen atom to which they are bonded may form a five- or six-membered ring.

3. Process according to Claim 1, **characterized in that**
R¹ is selected from the group comprising H, CH₃, CH₂COO- (C₁₋₁₂) -alkyl, and R² and R³ are each independently selected from the group comprising CF₃, CF₂H, CF₂Cl;
R⁴ is selected from the group comprising COOH, (C=O)OR⁵.

4. Compound of the formula (VI) 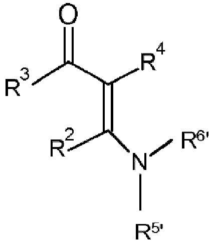 in which
R² and R³ are each independently selected from the group comprising CF₃, CF₂H, CF₂Cl;
R⁴ is selected from the group comprising (C=O)OR⁵;
R⁵' and R⁶' are each independently selected from the group comprising C₁₋₆-alkyl.

## Revendications

1. Procédé de fabrication de 3,5-bis(fluoroalkyl)-pyrazoles de formule (Ia) et (Ib) 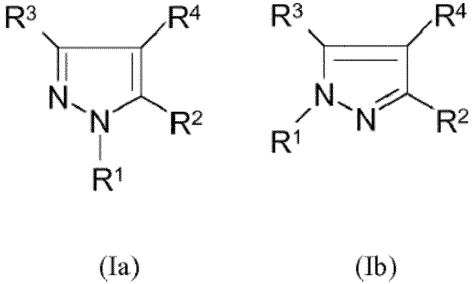 dans lesquelles
R¹ est choisi dans le groupe contenant H, alkyle en C₁₋₁₂, cycloalkyle en C₃₋₈, aryle en C₆₋₁₈, arylalkyle en C₇₋₁₉ ou alkylaryle en C₇₋₁₉, CH₂CN, CH₂CX₃, CH₂COOH, CH₂COO-alkyle en C₁₋₁₂,
les X représentant indépendamment les uns des autres F, Cl, Br, I ;
R² et R³ sont choisis indépendamment l'un de l'autre parmi les groupes halogénoalkyle en C₁-C₆ ;
R⁴ est choisi dans le groupe contenant Hal, COOH, (C=O)OR⁵, CN et (C=O)NR⁵R⁶, R⁵ et R⁶ étant choisis indépendamment l'un de l'autre dans le groupe contenant alkyle en C₁₋₁₂, cycloalkyle en C₃₋₈, aryle en C₆₋₁₈, arylalkyle en C₇₋₁₉ ou alkylaryle en C₇₋₁₉, ou R⁵ et R⁶ pouvant former ensemble avec l'atome N auquel ils sont reliés un cycle à cinq ou six éléments ;
**caractérisé en ce qu'**à l'étape A), la réaction d'α,α-dihalogénoamines de formule (II) 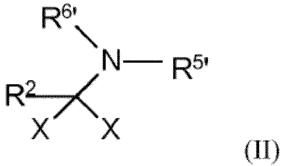 dans laquelle X représente Cl ou F, et R^{5'} et R^{6'} sont choisis indépendamment l'un de l'autre dans le groupe contenant alkyle en C₁₋₆, avec des composés de formule (III) 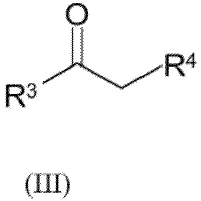 dans laquelle les radicaux R² et R³ ont la signification indiquée précédemment, a lieu et, à l'étape B), la réaction avec des hydrazines de formule (IV) 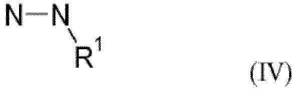 dans laquelle R¹ a la signification indiquée précédemment, a lieu.

2. Procédé selon la revendication 1, **caractérisé en ce que**
R¹ est choisi dans le groupe contenant H, alkyle en C₁₋₁₂, CH₂CN, CH₂COO-alkyle en C₁₋₁₂, et
R² et R³ sont choisis indépendamment l'un de l'autre dans le groupe contenant CF₃, CF₂H, CF₂Cl ;
R⁴ est choisi dans le groupe contenant COOH, (C=O)OR⁵, CN et (C=O)NR⁵R⁶, R⁵ et R⁶ étant choisis indépendamment l'un de l'autre dans le groupe contenant alkyle en C₁₋₁₂, cycloalkyle en C₃₋₈, aryle en C₆₋₁₈, arylalkyle en C₇₋₁₉ ou alkylaryle en C₇₋₁₉, ou R⁵ et R⁶ pouvant former ensemble avec l'atome N auquel ils sont reliés un cycle à cinq ou six éléments.

3. Procédé selon la revendication 1, caractérisé en c que
R¹ est choisi dans le groupe contenant H, CH₃, CH₂COO-alkyle en C₁₋₁₂, et
R² et R³ sont choisis indépendamment l'un de l'autre dans le groupe contenant CF₃, CF₂H, CF₂Cl ;
R⁴ est choisi dans le groupe contenant COOH, (C=O)OR⁵.

4. Composé de formule (VI) 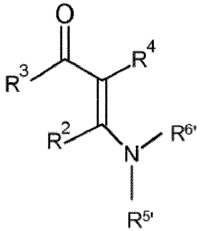 dans laquelle
R² et R³ sont choisis indépendamment l'un de l'autre dans le groupe contenant CF₃, CF₂H, CF₂Cl ;
R⁴ est choisi dans le groupe contenant (C=O)OR⁵ ;
R^{5'} et R^{6'} sont choisis indépendamment l'un de l'autre dans le groupe contenant alkyle en C₁₋₆.
